# EUROPEAN PATENT SPECIFICATION

(11) **EP 4 172 624 B1**
(45) Date of publication and mention of the grant of the patent: **15.07.2026**
(21) Application number: 21735295.4
(22) Date of filing: 24.06.2021
(51) Int. Cl.: G01N 33/543, G01N 33/68

(54) **METHOD FOR MEASURING THE AMOUNT OF TARGET PROTEINS**
METHODE ZUR MESSUNG DER MENGE AN ZIELPROTEINEN
PROCÉDÉ DE MESURE DE LA QUANTITÉ DE PROTÉINES CIBLES

(30) Priority: 26.06.2020 EP 20182679
(43) Date of publication of application: 03.05.2023
(73) Proprietor: ProteomEdge AB, 106 91 Stockholm (SE)
(72) Inventor: EDFORS, Fredrik, 113 46 Stockholm (SE); UHLÉN, Mathias, 181 90 Lidingö (SE)
(74) Representative: AWA Sweden AB
(86) International application number: PCT/EP2021/067375
(87) International publication number: WO 2021/260128

(56) References cited:
- WO-A1-2019/168843
- US-A1- 2015 253 341
- BOSTRÖM TOVE ET AL: "Antibodies as means for selective mass spectrometry", JOURNAL OF CHROMATOGRAPHY B, ELSEVIER, AMSTERDAM, NL, vol. 1021, 31 October 2015 (2015-10-31), pages 3 - 13, XP029535426, ISSN: 1570-0232, DOI: 10.1016/J.JCHROMB.2015.10.042

## Description

### Technical field

The present disclosure relates to a method for measuring the amount of target proteins in body fluid using an isotope-labelled standard protein by means of mass spectrometry.

### Background

Measurement of protein levels in body fluid is an essential component of assessing the health state of an individual. A large number of proteomics technologies have successfully been established and implemented into clinical practice, and are capable of providing information describing patients at the molecular level. More than one hundred clinical protein assays have been approved by the US Food and Drug Administration (FDA) for use in serum or plasma, and an equally large number of targets have been cleared for standardized laboratory tests in the US.

The antibody-based enzyme-linked immunosorbent assay (ELISA) is considered as the gold standard for quantitation of soluble proteins. ELISA provides rapid and robust results capable of sample analysis in high-throughput. However, this and other antibody-based assays are often limited to analysis of a single target protein or a limited number of target proteins (single-plex or low-plex assays). The reason may be cross-talk between probes, especially when using colorimetric read-out.

On the other hand, mass spectrometry (MS) technologies are capable of simultaneous analysis of a plurality of target proteins (multiplex), due to the high speed of the detector and the separation by mass. This is especially true when MS is used together with liquid chromatographic separation of proteins or peptides (LC-MS). The read-out, in combination with the use of affinity capture, can efficiently compensate for any selectivity biases introduced by antibodies in e.g. ELISA when quantifying proteins from a complex matrix.

An example of a technology for quantifying proteins by MS is the SISCAPA technology ("Stable Isotope Standards and Capture by Anti-Peptide Antibodies"). The technology uses a synthetic stable isotope labelled peptide as standard. A sample comprising target protein is digested using a proteolytic enzyme, such as trypsin. An antibody is then used to capture and thus enrich peptides of the target protein and a stable isotope labeled standard of that target protein. The natural (sample derived) and internal standard (isotope labeled) peptides are quantitated by LC-MS/MS, and their measured abundance ratio is used to calculate the abundance of the target protein.

Stable Isotope Labeling with Amino acids in Cell culture (SILAC) is a technique based on MS that detects differences in protein abundance among samples using isotopic labeling. In short, cells are differentially isotopically labeled by growing them in light vs. heavy medium. A heavy medium comprises isotopic labelling of one of the amino acids therein. Samples of heavy vs. light medium grown cells are combined, and a mass spectrometer is able to distinguish between the different isotope labelled proteins. An advantage of this technology is that this methodology is very accurate and introduces very low quantitative bias due to spiking of multiple versions of the next to identical proteomes. However, the method is not feasible for plasma or serum as no equivalent standard matrix is available.

Kim et al (Clinical Chemistry 64(8):1230-1238, 2018) teaches that a target protein can be quantified via the addition of a synthetic, isotopically labelled version of that protein. This article discloses a serum sample comprising a target protein and a stable isotope-labeled internal standard protein analog thereof, which is subjected to antibody enrichment and subsequent tryptic digestion. The digested sample may be analyzed using MS.

US2011/0143379 relates to a method for detecting both full-length thioredoxin as well as a truncated form thereof (a naturally occurring cleavage product) in a sample. The protein and its cleavage product may be identified by an antibody that recognizes a 7 amino acid long sequence shared between the two forms. The sample may be digested and analyzed by for example MS.

Boström et al (J Chrom B 1021:3-13, 2016) relates to enrichment of target protein and detection thereof in mass spectroscopy. The article discloses immunoenrichment prior to trypsin cleavage.

Although several methods for determination of proteins in body fluid exist, accurate determination of protein concentration in complex mixtures often suffers from inherent bias in and/or inefficiency of the methods used. There is still a need for improved accuracy and effectiveness. In other words, there is a call for new methods for measuring the amount of a target protein in body fluid.

### Disclosure of the invention

It is an object of the present invention to at least partly reduce or overcome challenges in the prior art, and provide means for accurate measurement of an amount of a target protein in body fluid, the measurement being on more than one target protein in the same sample, the sample being a sample from body fluid.

This and other objects, which will be apparent to a skilled person from the present disclosure, are achieved by the different aspects of the disclosure as defined in the appended claims and as generally disclosed herein. The invention is defined by the appended claims.

In a first aspect of the disclosure, there is provided a method for measuring the amount of a target protein in body fluid. The method comprises the initial step of preparing a sample suspected to comprise a target protein and adding a known amount of an isotope-labelled internal standard protein. The standard protein consists of a fragment of the target protein. Furthermore, the method comprises a step of bringing the prepared sample into contact with a solid support comprising a binding agent. The solid support is then washed in order to remove unbound members of the sample. Remaining target protein and standard protein are thereafter digested, providing a digested sample. After digestion, the digested sample is subjected to mass spectrometry, whereafter the amount of target protein in the sample can be determined by comparing with the standard protein. In the method as disclosed herein, the binding agent is capable of binding an epitope present in both the target protein and the standard protein. The measuring comprises measuring the amount of at least two target proteins, such as three target proteins, such as four target proteins, such as five target proteins, such as six target proteins, such as seven target proteins, such as eight target proteins, such as nine target proteins, such as ten target proteins. A plurality of target proteins are measured in parallel.

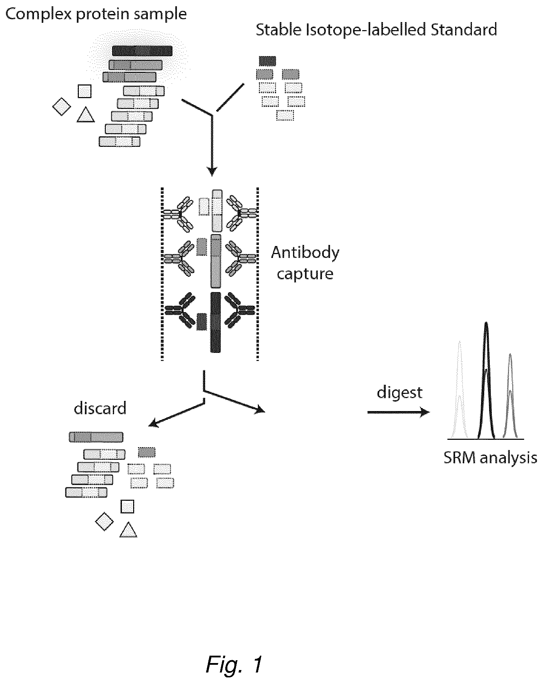

An embodiment of the first aspect of the disclosure is illustrated in Figure 1, which shows an overview of an affinity co-capture strategy according to one embodiment of this aspect. Here, a complex sample protein mixture is combined with a multiplex mixture of stable isotope labeled standard proteins. The standard proteins are spiked into the sample to quantify the endogenous protein level. A set of binding agents with affinity towards both the endogenous protein and its corresponding standard protein fragment is used to co-capture the protein targets. Non-binding proteins are discarded. Proteins are digested and quantified by LC-MS/MS. The relative protein amount can be quantified by knowing the spiked amount of spiked standard protein, after adjusting for differences in binding efficiency during the affinity capture step.

The method of the present disclosure is based on co-capture and digestion of a target protein together with its corresponding stable isotope-labelled standard protein. As disclosed herein, the target protein and its corresponding standard protein are captured simultaneously, i.e. co-captured, using the same binding agent. As the proteins are treated the same in all steps, there is no inherent bias of the method.

In the method disclosed herein, the capture step precedes the digestion step. An advantage of the capture occurring first is that no capture related bias can occur from incomplete digestion of the sample. Conversely, when digestion of a sample precedes the capture step, kinetic parameters of the entity digesting the sample may be of importance. For example, it may be difficult to obtain a complete digestion of the proteins in the sample when a proteolytic enzyme is used before capture. This may be due to different factors, such as limited diffusion, impaired enzyme activity, precipitation or aggregation of components in the sample, chemical modifications introduced by the enzyme (e.g. deamidation), and/or mis-cleavages (e.g. trimmed ends). Another risk is that the sample is over-digested if an enzyme with inferior specificity is used, in particular when incubation is prolonged or if digestion conditions, e.g. pH or temperature, are not optimal for that specific enzyme. That is, the proteolytic enzyme may cleave unspecifically at protein sites in the sample which do not correspond to an actual cleavage site for that enzyme.

When digesting proteins in a sample, the three dimensional folding structure of the digested proteins may disappear. An advantage of the capture step preceding the digestion step is that binding agents directed towards folded proteins may be used. For example, it is well known in the art that high quality antibodies towards peptides may be difficult to generate, as compared to generation of antibodies towards undigested proteins. The reason may be that the latter may maintain folded structures while the former may not, or may not maintain a three-dimensional structure to the same extent. The provision of antibodies towards folded structures may enable increased accuracy due to e.g. satisfactory binding kinetics.

The method of the present disclosure uses a labeled standard protein, which is added at a known amount. The standard protein is a fragment of the target protein, as discussed above. Because it is identical to the target protein over the length of the standard protein, except in a label, which label preferably is in the form of an isotope, the ratio between the standard protein and the target protein can be detected by MS. This enables the concentration of the natural target protein in the sample to be calculated.

Another advantage of the present disclosure is that the use of a binding agent for capturing target protein in the sample before digestion enables determination of the quantity of low abundant target proteins in a sample, since low abundant proteins can be enriched in the capturing step.

A related advantage is that when the sample comprises high abundant target proteins, determination of the quantity of the high abundant target proteins is enabled because the number of binding agents can be adjusted to capture a fraction of the high abundant proteins present. This concept is discussed further below.

When a sample is brought into contact with a binding agent, unbound members of the sample may be depleted by washing, resulting in a less complex sample. A further advantage of using the binding agent in a capture step before digestion in this way is that it allows higher sensitivity in the analysis, because background complexity in the sample is decreased. Another effect of having a less complex sample is that a lower amount of a digestive entity, such as a proteolytic enzyme, needs to be used.

Yet another advantage of using the binding agent in a capture step before digestion is that this may enable a faster analysis time due to an increased purity of the sample.

In some embodiments, the target protein is a soluble protein. In some embodiments, the target protein is a tissue specific biomarker. Non-limiting examples of tissue specific biomarker are prostate specific antigen (KLK3) or troponin originating from the heart. In preferred embodiments, the target protein is a water soluble protein. As used herein, water soluble proteins are defined as proteins that are at least partly soluble in water, as apparent to a person of skill in the art. In some embodiments, the target protein is a leakage product. Non-limiting examples of leakage products are highly abundant proteins such as histones; vimentin; or enzymes such as aspartate transaminase, prostate specific antigen (KLK3) or troponin originating from the heart. In some embodiments, the target protein is an actively secreted protein, i.e. a secretory protein that is secreted by a cell. In some embodiments, the secretory protein is a protein secreted into blood. Non-limiting examples of a target protein that may be secreted into blood are presented in Table 1. In some embodiments, the target protein is an FDA qualified biomarker. At the time of filing, a non-limiting list of FDA qualified biomarkers could be found at The U.S. Food and Drug Administration website, "List of Qualified Biomarkers" [retrieved on 2020-02-28]. Retrieved from <https://www.fda.gov/drugs/cder-biomarker-qualification-program/list-qualified-biomarkers>

In some embodiments, the target protein is selected from the group consisting of a cytokine, a chemokine, an interleukin, an interferon, a hormone, a neuropeptide, a growth factor, a receptor, a protein involved in transport, a protein involved in development, an enzyme, an enzyme inhibitor, a protein involved in the immune system, a protein involved in coagulation, a protein involved in the complement pathway, an acute phase protein and a cell adhesion protein.

In some embodiments, the solid support is a bead, such as a magnetic bead, or a column. Other types of solid supports are also possible, as apparent to the person of skill in the art. The binding agent may be attached to the solid support using techniques known to a person of skill in the art. According to the method disclosed herein, a sample suspected of comprising the target protein and its corresponding standard protein is brought into contact with a solid support. The contact enables binding of any target protein and its corresponding standard protein to the support via the binding agent. Due to the specificity of the binding agent, the remaining members of the sample do not bind, or bind only to a lesser extent. Any unbound members are discarded in a subsequent washing step, resulting in a higher purity of the sample.

The amount of target protein and its corresponding standard protein being captured can be adjusted by modifying the amount of binding agents used in the capturing step. For example, when capturing a high abundant target protein, all target proteins may not be bound by the binding agent. Thus, any unbound target protein and corresponding standard protein are discarded in a subsequent washing step. This results in a reduced amount of high abundant target protein and its corresponding standard protein in the captured sample. Moreover, when at least two target proteins of different abundance are to be quantified, the amount of binding agent used can be adjusted to compensate for this difference. This concept is further discussed below.

According to the method as disclosed herein, a sample comprising the at least one target protein and the at least one standard protein is digested. The digestion occurs after the washing step. In the digestion step, any captured material present on the solid support may be cleaved off.

Several different techniques for digesting proteins or peptides into smaller entities are contemplated, and would be apparent to the person of skill in the art. In some embodiments, the sample may be chemically digested. In other embodiments, the sample may be digested by means of a biological molecule, such as an enzyme. In preferred embodiments, the digestion is carried out by means of a proteolytic enzyme. The proteolytic enzyme may for example be trypsin.

In order to provide for accurate measurement of the digested sample, which comprises digested fragments of the target proteins and digested fragments of the standard protein, the digestion may be carried out such that the digested sample comprises at least one isotopically labeled standard peptide from the isotopically labeled standard protein, said peptide consisting of between 6 and 25 amino acids.

It is well known in the art how to produce a protein. A protein may for example be produced by means of recombinant DNA technology, or may be produced by means of a peptide synthesizer. In some embodiments, the standard protein is a recombinant protein. In other embodiments, the standard protein is a synthetic protein.

In some embodiments, the standard protein comprises a cleavage site for a proteolytic enzyme. In some embodiments, the standard protein comprises at least two cleavage sites for a proteolytic enzyme. In some embodiments, the standard protein comprises at least three cleavage sites for a proteolytic enzyme. An advantage of having one or more cleavage sites for a proteolytic enzyme is that multiple peptides may be used for protein quantitation, which may improve accuracy and/or precision. This can also help increase the quantitative accuracy across the protein sequence, based on sequence coverage, as it is known by persons of skill in the art that longer sequences provide higher accuracy in general.

As discussed above, the target protein and a corresponding standard protein may be distinguished from one another by at least one isotopically labelled amino acid in the standard protein. In some embodiments, the standard protein is labelled with at least one isotope selected from the group consisting of ¹⁵N, ¹³C and/or ¹⁸O. In another embodiment, the standard protein is labelled with deuterium. As apparent to the person of skill in the art, incorporation of other isotopes is also possible.

In some embodiments, the binding agent is an antibody or an antibody fragment. In some embodiments, the antibody or antibody fragment is a monoclonal antibody or fragment thereof. In some embodiments, the antibody or antibody fragment is a polyclonal antibody or fragment thereof. In some embodiments, the antibody fragment is an scFv. In some embodiments, the binding agent is an antibody mimetic. The binding agent may be produced and developed as disclosed in the appended examples.

The method as disclosed herein is used to measure a plurality of target proteins in parallel (multiplex mode). An advantage of measuring several proteins together is that the result may give a more accurate basis for providing a diagnosis, when several proteins are associated with the particular diagnosis. The measurement of target protein comprises measuring the amount of at least two target proteins, such as three target proteins, such as four target proteins, such as five target proteins, such as six target proteins, such as seven target proteins, such as eight target proteins, such as nine target proteins, such as ten target proteins. As apparent by the person of skill in the art, the method as disclosed herein may enable measurement of at least 20 target proteins, of at least 30 target proteins, of at least 40 target proteins, of at least 50 target proteins, of at least 60 target proteins, of at least 70 target proteins, of at least 80 target proteins, of at least 90 target proteins, or of at least 100 target proteins. In order to measure more than one target protein, more than one binding agent may be used in the step of capture. It is preferred that for each target protein, a corresponding binding agent is used. For example, when measuring 10 target proteins, 10 binding agents may be used. The corresponding binding agent may bind a target protein selectively. It is preferred that the binding agent does not bind other target proteins, or only does so with low affinity.

An advantage of capturing the sample is that this step may allow the dynamic range of highly and lowly abundant proteins to be adjusted. In some embodiments, at least two target proteins are present in the sample at a relative concentration of at least 10X in difference, such as 100X in difference, such as 1000X in difference, such as 10 000X in difference, such as 100 000X in difference, such as 1000 000X in difference, such as 10 000 000X in difference. For example, when a first target protein is present in low amounts in the sample while a second target protein is present in higher amounts in that sample, one may adjust the amount of binding agents used so that a greater number of binding agents are used for the low abundant target protein and a smaller number of binding agents are used for the high abundant target protein. This may provide a sample in which substantially all of the low abundant target protein is captured while only a certain fraction of the high abundant target protein is captured. In this way, the sample may, after the capture step, comprise amounts of the first and the second target proteins, i.e. here the low and high abundant target proteins respectively, that are within the same range, or at least closer in range, when subjected to mass spectrometry. This can be seen as the low abundant target being enriched while the amount of the high abundant target is reduced.

Another advantage of adjusting the amount of binding agent in this and related embodiments is that the sample may not be need to be diluted before mass spectrometry, which is otherwise usually the case for samples comprising high abundant proteins. As apparent to persons of skill in the art, needing to dilute a sample comprising a low abundant protein for the purpose of reducing the concentration of high abundant protein also present, may lead to such a reduction in the content of the low abundant protein that it may practically disappear.

Adjusting the amount of high and low abundant proteins in this way enables analysis of the sample with increased accuracy, because the concentrations of target proteins can be brought to be within the same range. Adjustment of the amount of binding proteins is also plausible when the at least two target proteins are three target proteins, such as four target proteins, such as five target proteins, such as six target proteins, such as seven target proteins, such as eight target proteins, such as nine target proteins, such as ten target proteins. Furthermore, one may adjust the number of different binding proteins to be added when measuring an amount of at least 20 target proteins, such as at least 30 target proteins, such as at least 40 target proteins, such as at least 50 target proteins, such as at least 60 target proteins, such as at least 70 target proteins, such as at least 80 target proteins, such as at least 90 target proteins, such as at least 100 target proteins, enabling analysis of the sample when the concentrations of target proteins are within the same range. As used herein, the phrase "within the same range" is an approximate description of an amount, allowing relative concentrations of for example 1:1, up to 5:1, up to 10:1, up to 20:1, up to 30:1, up to 40:1, up to 50:1, up to 60:1, up to 70:1, up to 80:1, up to 90:1, up to 100:1, up to 1000 :1, of a first target protein and a second target protein.

In some embodiments, the at least one target protein suspected to be present in the sample, is present in a concentration of between 10⁻⁴ and 10⁻¹⁰ M, such as between 10⁻⁶ and 10⁻⁷ M. An advantage of using the method according to the present disclosure is that the use of a binding agent for capture before digestion enables accurate determination of a target protein which is present in very small amounts in a sample. In some embodiments, the binding agent is present in substantial excess over the sum of the target protein and the corresponding standard protein. Thus, the highest possible amount of bound target protein and bound corresponding standard protein may be achieved.

In some embodiments, the binding agent binds the target protein and the corresponding standard protein with different affinities. The difference in affinity will lead to an established ratio in which the target protein and the corresponding standard protein are present after the capture step. This ratio may be maintained throughout the remaining steps of the method. One may account for the ratio when determining the amount of target protein by comparing with the standard protein.

In some embodiments, the target protein and the standard protein bind to the binding agent with comparable affinity, at a ratio of K_{D} values such as 1:1, such as 1:2, such as 1:3, such as 1:4, such as 1:5, such as 1:6, such as 1:7, such as 1:8, such as 1:9, such as 1:10, such as 1:11, such as 1:12, such as 1:13, such as 1:14, such as 1:15.

In order for the invention to work, the binding protein epitope must be shared between the target protein and its corresponding standard protein. That is, the target protein and the corresponding standard protein comprise the same epitope, and the binding agent recognizes this epitope when present on the target protein as well as when present on the corresponding standard protein. In some embodiments, the epitope comprises at least 4 amino acids, such as 5 amino acids, such as 6 amino acids, such as 7 amino acids, such as 8 amino acids, such as 9 amino acids, such as 10 amino acids, such as 11 amino acids, such as 12 amino acids, such as 13 amino acids, such as 14 amino acids, such as 15 amino acids, or more. In some embodiments, the epitope is linear. In other embodiments, the epitope is a conformational epitope.

In some embodiments, the body fluid is selected from the group consisting of plasma, serum, cerebrospinal fluid, urine, dry blood spots and saliva. In some embodiments, the body fluid is from a mammal. In some embodiments, the mammal is a human.

In some embodiments, the method is preceded by a step of approximation of the amount of target protein by establishing a standard curve, such as a forward standard curve or a reverse standard curve. An advantage of performing an approximation step is that one can estimate the amount of target protein in a sample, and adjust the addition of a standard protein to an amount within the same range as the amount of target protein. A definition of "within the same range" is given elsewhere herein. When at least two target proteins are to be measured, the amounts of the binding agents may be adjusted, as discussed above. Another advantage of performing an approximation of the amount of target protein in a sample, is that such approximation may enable further optional steps of ensuring that all the peptides are within a mass spectrometer's dynamic range and of using this dynamic range optimally. In some embodiments, the affinity of the binding agent for the target protein and its corresponding standard protein may differ. In those embodiments, the affinity of the binding agent to the full length target protein and for the full length standard protein may be measured to establish a ratio between the two affinities. The ratio may be used for determining a suitable amount of a standard protein to be added to a sample, in order to enable an outcome within the same range when carrying out a mass spectrometry analysis.

In some embodiments, the step of digesting the target protein and the standard protein is preceded by a step of eluting said target protein and said standard protein. An advantage of eluting the target proteins and their corresponding standard proteins before digestion is that the solid support may then be reused in another experiment.

In some embodiments, the step of subjecting the digested sample to mass spectrometry is preceded by a step of liquid chromatography of the digested sample. A step of liquid chromatography may aid in obtaining a purer sample or a less complex sample. Liquid chromatography may separate fractions of a sample in terms of size, level of hydrophobicity, electrostatic attraction, or by affinity for a functional group. After separation, fractions of the sample that comprise target protein are selected and fractions that do not are discarded, thereby improving sample purity.

In some embodiments, the standard protein is added to the sample in an amount approximately equal to the amount of the target protein suspected to be present in that sample. An advantage of providing a sample wherein a target protein and a corresponding standard protein are present in approximately equal amounts may be that an MS reading within the same range is enabled. As understood by the person of skill in the art, approximately equal amounts of a target protein and a corresponding standard protein is understood to mean within the same order of magnitude or within the same range. For example, the approximately equal amounts may be at a ratio of target protein:standard protein of 1:10, such as 1:9, such as 1:8, such as 1:7, such as 1:6, such as 1:5, such as 1:4, such as 1:3, such as 1:2, such as 1:1, such as 2:1, such as 3:1, such as 4:1, such as 5:1, such as 6:1, such as 7:1, such as 8:1, such as 9:1, such as 10:1.

In some embodiments, the type of mass spectrometry used is tandem mass spectrometry with data dependent acquisition mode. In other embodiments, the mass spectrometry used is tandem mass spectrometry with data independent acquisition mode. In yet other embodiments, the mass spectrometry used is tandem mass spectrometry with selective reaction monitoring mode. As apparent for a person of skill in the art, other types of mass spectrometry methods are also possible to use. The mass analyzer of the mass spectrometry instrument may be an ion trap, a triple quadrupole, an ESI-TOF, a Q-TOF type instrument, an orbitrap, or any other instrument of suitable mass resolution (> 1,000) and sensitivity.

As apparent to the person of skill in the art, any target protein in a sample may be analyzed according to the first aspect of the present disclosure. The following is a non-limiting list of possible target proteins, present in human plasma and secreted into blood **(Table 1).**

**Table 1. Proteins present in human plasma and secreted into blood.**

| **Target proteins of the complement pathway** |
|---|
| complement C4A (Rodgers blood group) |
| complement C3 |
| complement C5 |
| complement C1s |
| complement C1r |
| complement C1q A chain |
| complement C1q C chain |
| complement C1q B chain |
| mannose binding lectin 2 |
| complement C2 |
| complement C8 gamma chain |
| complement C8 alpha chain |
| complement C8 beta chain |
| complement C9 |
| complement C7 |
| complement C6 |
| clusterin |
| mannan binding lectin serine peptidase 2 |
| complement component 4 binding protein beta |
| complement component 4 binding protein alpha |
| C1q and TNF related 1 |
| C1q and TNF related 12 |
| C1q and TNF related 2 |
| C1q and TNF related 3 |
| C1q and TNF related 5 |
| C1q and TNF related 6 |
| C1q and TNF related 7 |
| C1q and TNF related 9 |
| complement C1r subcomponent like |
| complement C4B (Chido blood group) |
| CD55 molecule (Cromer blood group) |
| complement factor H related 1 |
| complement factor H related 2 |
| complement factor H related 3 |
| complement factor H related 4 |
| complement factor H related 5 |
| complement factor I |

| **Target proteins of acute phase** |
|---|
| fibronectin 1 |
| orosomucoid 1 |
| orosomucoid 2 |
| serpin family F member 2 |
| C-reactive protein |
| haptoglobin |
| interleukin 6 |
| serpin family A member 3 |
| inter-alpha-trypsin inhibitor heavy chain family member 4 |
| CD163 molecule |
| serum amyloid A1 |
| serum amyloid A2 |
| serum amyloid A4, constitutive |

| **Chemokine target proteins** |
|---|
| C-X-C motif chemokine ligand 8 |
| C-C motif chemokine ligand 2 |
| C-C motif chemokine ligand 1 |
| C-C motif chemokine ligand 11 |
| C-C motif chemokine ligand 13 |
| C-C motif chemokine ligand 14 |
| C-C motif chemokine ligand 15 |
| C-C motif chemokine ligand 16 |
| C-C motif chemokine ligand 17 |
| C-C motif chemokine ligand 18 |
| C-C motif chemokine ligand 19 |
| C-C motif chemokine ligand 20 |
| C-C motif chemokine ligand 21 |
| C-C motif chemokine ligand 22 |
| C-C motif chemokine ligand 23 |
| C-C motif chemokine ligand 24 |
| C-C motif chemokine ligand 25 |
| C-C motif chemokine ligand 26 |
| C-C motif chemokine ligand 27 |
| C-C motif chemokine ligand 28 |
| C-C motif chemokine ligand 3 |
| C-C motif chemokine ligand 3 like 1 |
| C-C motif chemokine ligand 4 |
| C-C motif chemokine ligand 4 like 2 |
| C-C motif chemokine ligand 5 |
| C-C motif chemokine ligand 7 |
| C-C motif chemokine ligand 8 |
| C-X3-C motif chemokine ligand 1 |
| C-X-C motif chemokine ligand 1 |
| C-X-C motif chemokine ligand 10 |
| C-X-C motif chemokine ligand 11 |
| C-X-C motif chemokine ligand 12 |
| C-X-C motif chemokine ligand 13 |
| C-X-C motif chemokine ligand 14 |
| C-X-C motif chemokine ligand 16 |
| C-X-C motif chemokine ligand 2 |
| C-X-C motif chemokine ligand 3 |
| C-X-C motif chemokine ligand 5 |
| C-X-C motif chemokine ligand 6 |
| C-X-C motif chemokine ligand 9 |
| X-C motif chemokine ligand 1 |
| X-C motif chemokine ligand 2 |

| **Interleukin target proteins** |
|---|
| interleukin 11 |
| interleukin 1 alpha |
| interleukin 1 beta |
| interleukin 2 |
| interleukin 15 |
| interleukin 18 |
| interleukin 7 |
| interleukin 10 |
| interleukin 3 |
| interleukin 5 |
| interleukin 13 |
| interleukin 4 |
| interleukin 9 |
| interleukin 12B |
| interleukin 12A |
| interleukin 16 |
| interleukin 17A |
| interleukin 17B |
| interleukin 17C |
| interleukin 17D |
| interleukin 17F |
| interleukin 19 |
| interleukin 1 family member 10 |
| interleukin 20 |
| interleukin 21 |
| interleukin 22 |
| interleukin 23 subunit alpha |
| interleukin 24 |
| interleukin 25 |
| interleukin 26 |
| interleukin 27 |
| interleukin 31 |
| interleukin 32 |
| interleukin 33 |
| interleukin 34 |
| interleukin 36 alpha |
| interleukin 36 beta |
| interleukin 36 gamma |
| interleukin 36 receptor antagonist |
| interleukin 37 |

| **Interferon target proteins** |
|---|
| interferon gamma |
| interferon alpha 1 |
| interferon alpha 10 |
| interferon alpha 13 |
| interferon alpha 14 |
| interferon alpha 16 |
| interferon alpha 17 |
| interferon alpha 2 |
| interferon alpha 21 |
| interferon alpha 4 |
| interferon alpha 5 |
| interferon alpha 6 |
| interferon alpha 7 |
| interferon alpha 8 |
| interferon beta 1 |
| interferon epsilon |
| interferon kappa |
| interferon lambda 1 |
| interferon lambda 2 |
| interferon lambda 3 |
| interferon omega 1 |

| **Cytokine target proteins** |
|---|
| glucose-6-phosphate isomerase |
| pro-platelet basic protein |
| lymphotoxin alpha |
| tumor necrosis factor |
| colony stimulating factor 3 |
| colony stimulating factor 2 |
| aminoacyl tRNA synthetase complex interacting multifunctional protein 1 |
| bone morphogenetic protein 10 |
| bone morphogenetic protein 2 |
| bone morphogenetic protein 4 |
| bone morphogenetic protein 6 |
| bone morphogenetic protein 7 |
| bone morphogenetic protein 8a |
| bone morphogenetic protein 8b |
| chromosome 17 open reading frame 99 |
| CD40 ligand |
| chemokine like factor |
| cardiotrophin like cytokine factor 1 |
| colony stimulating factor 1 |
| cardiotrophin 1 |
| Epstein-Barr virus induced 3 |
| family with sequence similarity 3 member B |
| Fas ligand |
| growth differentiation factor 1 |
| growth differentiation factor 11 |
| growth differentiation factor 15 |
| growth differentiation factor 2 |
| growth differentiation factor 3 |
| growth differentiation factor 5 |
| growth differentiation factor 6 |
| gremlin 1, DAN family BMP antagonist |
| gremlin 2, DAN family BMP antagonist |
| granulin precursor |
| macrophage migration inhibitory factor |
| nicotinamide phosphoribosyltransferase |
| nodal growth differentiation factor |
| oncostatin M |
| platelet factor 4 |
| platelet factor 4 variant 1 |
| secretoglobin family 3A member 1 |
| secreted phosphoprotein 1 |
| thrombopoietin |
| TNF superfamily member 10 |
| TNF superfamily member 11 |
| TNF superfamily member 12 |
| TNF superfamily member 13 |
| TNF superfamily member 13b |
| TNF superfamily member 14 |
| TNF superfamily member 15 |
| thymic stromal lymphopoietin |
| V-set and transmembrane domain containing 1 |

| **Apolipoprotein target proteins** |
|---|
| apolipoprotein M |
| apolipoprotein B |
| apolipoprotein A1 |
| apolipoprotein L1 |
| apolipoprotein E |
| apolipoprotein C4 |
| apolipoprotein A4 |
| apolipoprotein F |
| apolipoprotein D |
| apolipoprotein H |
| apolipoprotein A2 |
| apolipoprotein A5 |
| apolipoprotein C1 |
| apolipoprotein C2 |
| apolipoprotein C3 |
| apolipoprotein L4 |
| apolipoprotein O |

| **Hormonal target proteins** |
|---|
| prolactin |
| glycoprotein hormones, alpha polypeptide |
| luteinizing hormone beta polypeptide |
| transthyretin |
| inhibin alpha subunit |
| insulin like growth factor 2 |
| insulin |
| parathyroid hormone |
| calcitonin related polypeptide alpha |
| follicle stimulating hormone beta subunit |
| growth hormone 1 |
| inhibin beta A subunit |
| erythropoietin |
| natriuretic peptide B |
| thyroid stimulating hormone beta |
| oxytocin/neurophysin I prepropeptide |
| arginine vasopressin |
| inhibin beta B subunit |
| gastric inhibitory polypeptide |
| cholecystokinin |
| adiponectin, C1Q and collagen domain containing |
| inhibin beta C subunit |
| adrenomedullin |
| adrenomedullin 2 |
| angiopoietin like 8 |
| calcitonin related polypeptide beta |
| chorionic gonadotropin beta subunit 1 |
| chorionic gonadotropin beta subunit 3 |
| chorionic gonadotropin beta subunit 5 |
| chorionic gonadotropin beta subunit 8 |
| coatomer protein complex subunit alpha |
| corticotropin releasing hormone |
| chorionic somatomammotropin hormone 1 |
| chorionic somatomammotropin hormone 2 |
| chorionic somatomammotropin hormone like 1 |
| erythroferrone |
| fibrillin 1 |
| galanin and GMAP prepropeptide |
| glucagon |
| growth hormone 2 |
| ghrelin and obestatin prepropeptide |
| gonadotropin releasing hormone 1 |
| gonadotropin releasing hormone 2 |
| glycoprotein hormone alpha 2 |
| glycoprotein hormone beta 5 |
| hepcidin antimicrobial peptide |
| islet amyloid polypeptide |
| inhibin beta E subunit |
| insulin like 3 |
| insulin like 4 |
| insulin like 5 |
| insulin like 6 |
| klotho |
| meteorin like, glial cell differentiation regulator |
| motilin |
| natriuretic peptide A |
| natriuretic peptide C |
| pro-melanin concentrating hormone |
| proopiomelanocortin |
| pancreatic polypeptide |
| peptide YY |
| resistin |
| resistin like beta |
| relaxin 1 |
| relaxin 2 |
| relaxin 3 |
| secretin |
| spexin hormone |
| somatostatin |
| torsin family 2 member A |
| urocortin |
| urocortin 2 |
| urocortin 3 |
| urotensin 2 |
| vasoactive intestinal peptide |

| **Target neuropeptides** |
|---|
| neuropeptide Y |
| CART prepropeptide |
| hypocretin neuropeptide precursor |
| neuropeptide W |
| proprotein convertase subtilisin/kexin type 1 inhibitor |
| prepronociceptin |
| prokineticin 2 |
| secretogranin V |
| tachykinin precursor 1 |

| **Target growth factors** |
|---|
| vascular endothelial growth factor A |
| transforming growth factor beta 1 |
| vascular endothelial growth factor C |
| vascular endothelial growth factor B |
| VGF nerve growth factor inducible |
| TIMP metallopeptidase inhibitor 1 |
| platelet derived growth factor C |
| anti-Mullerian hormone |
| brain derived neurotrophic factor |
| betacellulin |
| epiregulin |
| fibroblast growth factor 19 |
| fibroblast growth factor 21 |
| fibroblast growth factor 23 |
| heparin binding EGF like growth factor |
| hepatocyte growth factor |
| insulin like growth factor 1 |
| midkine |
| melanoma inhibitory activity |
| nephroblastoma overexpressed |
| neuregulin 1 |
| neuregulin 2 |
| neurturin |
| platelet derived growth factor subunit A |
| platelet derived growth factor subunit B |
| platelet derived growth factor D |
| placental growth factor |
| prokineticin 1 |
| pleiotrophin |
| transforming growth factor alpha |
| transforming growth factor beta 2 |
| transforming growth factor beta 3 |
| vascular endothelial growth factor D |

| **Target receptors** |
|---|
| transferrin receptor |
| epidermal growth factor receptor |
| TNF receptor superfamily member 1A |
| TNF receptor superfamily member 1B |
| growth hormone receptor |
| interleukin 1 receptor accessory protein |
| adhesion G protein-coupled receptor G1 |
| fms related tyrosine kinase 4 |
| angiotensin I converting enzyme 2 |
| adhesion G protein-coupled receptor E3 |
| adhesion G protein-coupled receptor E5 |
| attractin |
| CD40 molecule |
| colony stimulating factor 2 receptor alpha subunit |
| Fas cell surface death receptor |
| Fc fragment of IgE receptor II |
| Fc fragment of IgG receptor IIIa |
| Fc fragment of IgG receptor IIIb |
| Fc receptor like 5 |
| fms related tyrosine kinase 1 |
| folate receptor 1 |
| folate receptor beta |
| folate receptor 3 |
| interferon alpha and beta receptor subunit 2 |
| interleukin 15 receptor subunit alpha |
| interleukin 1 receptor type 1 |
| interleukin 1 receptor type 2 |
| interleukin 1 receptor like 1 |
| interleukin 22 receptor subunit alpha 2 |
| interleukin 4 receptor |
| interleukin 6 signal transducer |
| leptin receptor |
| leukocyte immunoglobulin like receptor A2 |
| leukocyte immunoglobulin like receptor A5 |
| MET proto-oncogene, receptor tyrosine kinase |
| paired immunoglobin like type 2 receptor alpha |
| phospholipase A2 receptor 1 |
| protein tyrosine kinase 7 (inactive) |
| signaling lymphocytic activation molecule family member 1 |
| sortilin related receptor 1 |
| TEK receptor tyrosine kinase |
| transforming growth factor beta receptor 3 |
| TNF receptor superfamily member 18 |
| TNF receptor superfamily member 6b |

| **Target proteins involved in transport** |
|---|
| serpin family A member 7 |
| retinol binding protein 4 |
| lipoprotein(a) |
| albumin |
| hemopexin |
| sex hormone binding globulin |
| transferrin |
| ceruloplasmin |
| serpin family A member 6 |
| GC, vitamin D binding protein |
| lipopolysaccharide binding protein |
| NPC intracellular cholesterol transporter 2 |
| serpin family A member 5 |
| afamin |
| prostaglandin D2 synthase |
| cholesteryl ester transfer protein |
| protein kinase domain containing, cytoplasmic |
| phospholipid transfer protein |
| transcobalamin 1 |
| transcobalamin 2 |

| **Target developmental proteins** |
|---|
| angiogenic factor with G-patch and FHA domains 1 |
| angiogenin |
| angiopoietin 1 |
| angiopoietin 2 |
| angiopoietin 4 |
| angiopoietin like 4 |
| angiopoietin like 6 |
| collectin subfamily member 11 |
| C-X-C motif chemokine ligand 17 |
| EGF like repeats and discoidin domains 3 |
| ephrin A1 |
| ER membrane protein complex subunit 10 |
| endothelial cell specific molecule 1 |
| matrix Gla protein |
| myeloid derived growth factor |
| noggin |
| notch 2 N-terminal like |
| signal peptide, CUB domain and EGF like domain containing 2 |
| semaphorin 3F |
| semaphorin 3G |
| thrombospondin type 1 domain containing 7A |

| **Target protein involved in defense** |
|---|
| chitinase 3 like 1 |
| azurocidin 1 |
| bactericidal/permeability-increasing protein |
| defensin alpha 4 |
| granulysin |
| liver enriched antimicrobial peptide 2 |

| **Target enzymes** |
|---|
| angiotensin I converting enzyme |
| plasminogen activator, tissue type |
| lysozyme |
| renin |
| biotinidase |
| acid phosphatase, prostate |
| butyrylcholinesterase |
| ribonuclease A family member 3 |
| glutathione peroxidase 3 |
| matrix metallopeptidase 9 |
| glycosylphosphatidylinositol specific phospholipase D1 |
| alpha-L-fucosidase 2 |
| HGF activator |
| cathepsin D |
| paraoxonase 3 |
| lecithin-cholesterol acyltransferase |
| adenosine deaminase 2 |
| hyaluronan binding protein 2 |
| ribonuclease T2 |
| paraoxonase 1 |
| NAD(P)HX epimerase |
| glutaminyl-peptide cyclotransferase |
| ectonucleoside triphosphate diphosphohydrolase 5 |
| ADAM metallopeptidase domain 28 |
| acyloxyacyl hydrolase |
| ADP-ribosyltransferase 4 (Dombrock blood group) |
| chitinase 3 like 2 |
| chitinase 1 |
| chymase 1 |
| carboxypeptidase A3 |
| carboxypeptidase N subunit 1 |
| carboxypeptidase Q |
| cathepsin B |
| cathepsin W |
| dehydrogenase/reductase X-linked |
| deoxyribonuclease 1 like 3 |
| elastase, neutrophil expressed |
| ectonucleoside triphosphate diphosphohydrolase 6 (putative) |
| glutathione peroxidase 7 |
| granzyme A |
| granzyme B |
| granzyme H |
| granzyme K |
| HtrA serine peptidase 1 |
| hyaluronoglucosaminidase 1 |
| hyaluronoglucosaminidase 3 |
| interleukin 4 induced 1 |
| kallikrein related peptidase 15 |
| lipase C, hepatic type |
| lipase G, endothelial type |
| lipase H |
| lipoprotein lipase |
| mannosidase alpha class 2B member 2 |
| matrix metallopeptidase 25 |
| proprotein convertase subtilisin/kexin type 5 |
| proprotein convertase subtilisin/kexin type 9 |
| phospholipase A2 group XIIA |
| phospholipase A2 group IIA |
| phospholipase A2 group III |
| phospholipase A2 group VII |
| paraoxonase 2 |
| peptidylprolyl isomerase A |
| peptidylprolyl isomerase B |
| serine protease 23 |
| serine protease 33 |
| serine protease 57 |
| proteinase 3 |
| ribonuclease A family member 2 |
| ribonuclease A family member 4 |
| ribonuclease A family member k6 |
| renalase, FAD dependent amine oxidase |
| serine carboxypeptidase 1 |
| sphingomyelin phosphodiesterase 1 |
| sphingomyelin phosphodiesterase acid like 3A |
| superoxide dismutase 3 |
| ST6 beta-galactoside alpha-2,6-sialyltransferase 1 |
| tryptase alpha/beta 1 |
| tryptase beta 2 (gene/pseudogene) |
| vasohibin 1 |
| vasohibin 2 |
| vanin 3 |
| xylosyltransferase 1 |
| xylosyltransferase 2 |

| **Target enzyme inhibitors** |
|---|
| cystatin C |
| serpin family E member 1 |
| alpha-2-macroglobulin |
| inter-alpha-trypsin inhibitor heavy chain 2 |
| peptidase inhibitor 16 |
| alpha-1-microglobulin/bikunin precursor |
| PZP, alpha-2-macroglobulin like |
| inter-alpha-trypsin inhibitor heavy chain 1 |
| inter-alpha-trypsin inhibitor heavy chain 3 |
| fetuin B |
| serpin family A member 4 |
| C3 and PZP like, alpha-2-macroglobulin domain containing 8 |
| cystatin F |
| histocompatibility minor serpin domain containing |
| inter-alpha-trypsin inhibitor heavy chain family member 6 |
| peptidase inhibitor 3 |
| serpin family A member 11 |
| serpin family A member 9 |
| serine peptidase inhibitor, Kazal type 2 |
| serine peptidase inhibitor, Kazal type 8 (putative) |
| serine peptidase inhibitor, Kunitz type 1 |

| **Target immunity proteins** |
|---|
| complement factor B |
| S100 calcium binding protein A9 |
| S100 calcium binding protein A8 |
| complement factor properdin |
| peptidoglycan recognition protein 2 |
| ficolin 3 |
| CD5 molecule like |
| complement factor H |
| CD14 molecule |
| mannan binding lectin serine peptidase 1 |
| annexin A1 |
| complement factor D |
| galectin 3 |
| endoplasmic reticulum aminopeptidase 1 |
| Immunoglobulin heavy variable 4-38-2 |
| CD6 molecule |
| endoplasmic reticulum aminopeptidase 2 |
| Fc fragment of IgM receptor |
| ficolin 1 |
| ficolin 2 |
| granzyme M |
| major histocompatibility complex, class I, G |
| ISG15 ubiquitin-like modifier |
| galectin 9 |
| lymphocyte antigen 86 |
| lymphocyte antigen 96 |
| peptidoglycan recognition protein 1 |
| progesterone immunomodulatory binding factor 1 |
| S100 calcium binding protein A12 |
| secretory leukocyte peptidase inhibitor |
| sphingomyelin phosphodiesterase acid like 3B |
| spondin 2 |

| **Target cell adhesion proteins** |
|---|
| vitronectin |
| insulin like growth factor binding protein acid labile subunit |
| transforming growth factor beta induced |
| insulin like growth factor binding protein 7 |
| angiopoietin like 3 |
| fibroblast activation protein alpha |
| junctional adhesion molecule 3 |
| galectin 3 binding protein |
| milk fat globule-EGF factor 8 protein |
| protocadherin 12 |
| spondin 1 |
| sushi repeat containing protein, X-linked 2 |
| TNF alpha induced protein 6 |
| **Other target proteins secreted into blood** |
| insulin like growth factor binding protein 1 |
| insulin like growth factor binding protein 3 |
| bone gamma-carboxyglutamate protein |
| alpha 2-HS glycoprotein |
| insulin like growth factor binding protein 2 |
| angiotensinogen |
| insulin like growth factor binding protein 5 |
| gelsolin |
| insulin like growth factor binding protein 4 |
| haptoglobin-related protein |
| retinoic acid receptor responder 2 |
| annexin A2 |
| chromosome 6 open reading frame 120 |
| collagen like tail subunit of asymmetric acetylcholinesterase |
| cellular repressor of E1A stimulated genes 1 |
| endothelin 1 |
| endothelin 2 |
| endothelin 3 |
| fibroblast growth factor binding protein 2 |
| follistatin like 1 |
| growth arrest specific 6 |
| insulin like growth factor binding protein 6 |
| leukocyte cell derived chemotaxin 2 |
| galectin 1 |
| latent transforming growth factor beta binding protein 3 |
| marginal zone B and B1 cell specific protein |
| nucleobindin 1 |
| R-spondin 3 |
| tachykinin 4 |

| **Target proteins secreted into blood with no annotated function** |
|---|
| alpha fetoprotein |
| interleukin 1 receptor antagonist |
| leptin |
| oncoprotein induced transcript 3 |
| leucine rich alpha-2-glycoprotein 1 |
| alpha-1-B glycoprotein |
| secretogranin II |
| joining chain of multimeric IgA and IgM |
| serpin family F member 1 |
| fibrinogen like 1 |
| alpha-2-glycoprotein 1, zinc-binding |
| C-type lectin domain family 3 member B |
| macrophage stimulating 1 |
| carboxypeptidase N subunit 2 |
| secreted protein acidic and cysteine rich |
| ABO, alpha 1-3-N-acetylgalactosaminyltransferase and alpha 1-3-galactosyltransferase |
| angiopoietin like 1 |
| angiopoietin like 2 |
| amyloid P component, serum |
| agouti signaling protein |
| the protein with the Gene ID BX248415.1 |
| chromosome 16 open reading frame 89 |
| chromosome 1 open reading frame 54 |
| chromosome 1 open reading frame 56 |
| chromosome 2 open reading frame 40 |
| chromosome 4 open reading frame 48 |
| cathelicidin antimicrobial peptide |
| coiled-coil domain containing 126 |
| coiled-coil domain containing 134 |
| coiled-coil domain containing 3 |
| C-type lectin domain family 18 member A |
| C-type lectin domain family 18 member B |
| C-type lectin domain family 18 member C |
| collectin subfamily member 10 |
| cysteine rich with EGF like domains 2 |
| corticotropin releasing hormone binding protein |
| cysteine rich secretory protein LCCL domain containing 2 |
| cartilage associated protein |
| cutA divalent cation tolerance homolog |
| ephrin A4 |
| energy homeostasis associated |
| endogenous retrovirus group MER34 member 1, envelope |
| family with sequence similarity 177 member A1 |
| fibrinogen like 2 |
| follistatin |
| follistatin like 4 |
| gastrin releasing peptide |
| guanylate cyclase activator 2A |
| guanylate cyclase activator 2B |
| insulin like growth factor binding protein like 1 |
| IgA inducing protein |
| immunoglobulin lambda like polypeptide 1 |
| immunoglobulin lambda like polypeptide 5 |
| interleukin 18 binding protein |
| inter-alpha-trypsin inhibitor heavy chain family member 5 |
| intelectin 1 |
| leucine rich repeat LGI family member 2 |
| mesencephalic astrocyte derived neurotrophic factor |
| methyltransferase like 9 |
| MT-RNR2-like 1 |
| MT-RNR2-like 10 |
| MT-RNR2-like 11 |
| MT-RNR2-like 12 |
| MT-RNR2-like 4 |
| MT-RNR2-like 6 |
| MT-RNR2-like 8 |
| neural EGFL like 2 |
| neuromedin B |
| neuronal pentraxin 2 |
| nucleobindin 2 |
| phospholipase A2 inhibitor and LY6/PLAUR domain containing |
| phospholipase A2 group XIIB |
| plasminogen-like B1 |
| plasminogen-like B2 |
| protease associated domain containing 1 |
| pentraxin 3 |
| ring finger and SPRY domain containing 1 |
| signal peptide, CUB domain and EGF like domain containing 1 |
| signal peptide, CUB domain and EGF like domain containing 3 |
| syndecan 1 |
| syndecan 4 |
| selenoprotein P |
| suppressor of glucose, autophagy associated 1 |
| SPARC/osteonectin, cwcv and kazal like domains proteoglycan 1 |
| SPARC/osteonectin, cwcv and kazal like domains proteoglycan 2 |
| secreted phosphoprotein 2 |
| small vasohibin binding protein |
| trefoil factor 3 |
| versican |
| vitelline membrane outer layer 1 homolog |
| WD repeat domain 25 |

| **Target proteins involved in coagulation** |
|---|
| coagulation factor IX |
| coagulation factor X |
| plasminogen activator, urokinase |
| coagulation factor XIII A chain |
| plasminogen |
| serpin family A member 1 |
| protein C, inactivator of coagulation factors Va and VIIIa |
| fibrinogen beta chain |
| fibrinogen alpha chain |
| fibrinogen gamma chain |
| serpin family G member 1 |
| von Willebrand factor |
| serpin family C member 1 |
| coagulation factor XIII B chain |
| protein S |
| coagulation factor VIII |
| coagulation factor VII |
| serpin family D member 1 |
| coagulation factor XI |
| ADAM metallopeptidase with thrombospondin type 1 motif 13 |
| coagulation factor II, thrombin |
| coagulation factor V |
| coagulation factor XII |
| kininogen 1 |
| carboxypeptidase B2 |
| serpin family A member 10 |
| kallikrein B1 |
| histidine rich glycoprotein |
| coagulation factor III, tissue factor |
| protein Z, vitamin K dependent plasma glycoprotein |
| tissue factor pathway inhibitor |

Disclosed but not claimed is a kit for carrying out the method according to the first aspect, in any one of the embodiments described herein. The kit comprises at least one binding agent, at least one isotope-labelled internal standard protein, and instructions for carrying out the method. The binding agent comprised in the kit is selected such that it binds a target protein and the standard protein with comparable affinity, at a ratio of K_{D} values such as 1:1, such as 1:2, such as 1:3, such as 1:4, such as 1:5, such as 1:6, such as 1:7, such as 1:8, such as 1:9, such as 1:10, such as 1:11, such as 1:12, such as 1:13, such as 1:14, such as 1:15. Disclosed but not claimed is that the binding agent of the kit may be a monoclonal antibody or that the binding agent may be an scFv fragment.

### Definitions

As used herein, a "fragment" of a protein means any part of a protein or polypeptide, which is not the full length of that protein or polypeptide.

As used herein, a "body fluid" may be any liquid of the mammalian body. Non-limiting examples are intravascular fluid, intracellular fluid, extracellular fluid, interstitial fluid, lymphatic fluid and transcellular fluid.

As used herein, "comparable affinity" is defined as affinity within the same range, such as within a ratio of within 1:100, preferably within a ratio of 1:10, when comparing K_{D}-values for a target protein and a corresponding standard protein. A comparable affinity may be for example at a ratio of K_{D} values such as 1:1, such as 1:2, such as 1:3, such as 1:4, such as 1:5, such as 1:6, such as 1:7, such as 1:8, such as 1:9, such as 1:10, such as 1:11, such as 1:12, such as 1:13, such as 1:14, such as 1:15. In order to enable readings within the same range for both the target protein and the added standard protein, the amount of added standard protein may be adjusted, as discussed above.

As used herein, a "binding agent" may be any molecule, such as a biological molecule, that binds to a target protein such that the target protein is captured when carrying out the method of the disclosure. The binding agent may do so in a specific manner, such that both a target protein and a corresponding standard protein are bound and captured. As non-limiting examples, a binding agent may be selected from the group consisting of proteins, polypeptides, peptides, nucleic acids (oligonucleotides and polynucleotides), antibodies, ligands, polysaccharides, microorganisms, receptors, antibiotics and synthetic organic compounds.

As used herein, an "antibody" may belong to any class of immunoglobulin molecules of any species, or be derived therefrom, or be another specific binding agent constructed by variation of a conserved molecular scaffold so as to specifically bind an analyte or target protein or fragment thereof. In general, any use made of an antibody is understood as a use that could also be made of a binding agent as defined above.

The term "bind" includes any physical attachment or close association, which may be permanent or temporary (i.e. reversible). Generally, reversible binding includes aspects of charge interactions, hydrogen bonding, hydrophobic forces, van der Waals forces etc., that facilitate physical attachment between the molecule of interest and the analyte being measured.

The terms "internal standard", "standard protein", standard peptide", "isotope-labeled or internal standard protein", each mean an altered version of a respective internal standard protein that 1) is recognized as equivalent to the target protein or a fragment thereof by the appropriate binding agent and 2) differs from the target protein or a fragment thereof in a manner that can be distinguished by a mass spectrometer. It is preferred that the target protein and its corresponding standard protein are distinguishable through direct measurement of molecular mass, where a difference lies in the use of isotopic labeling of the standard protein. The distinguishing may also be made through mass measurement of fragments (e.g., through MS/MS analysis), or by another equivalent means.

### Brief description of the drawings

Figure 1 illustrates an embodiment of the disclosed method. Schematically, it shows the co-capture and digestion of endogenous proteins together with their corresponding stable isotope-labelled protein standard, with subsequent binding agent capture, and digestion, followed by MS analysis.
Figure 2 illustrates surface plasmon resonance experiments on the indicated full-length target proteins (A-D) and standard proteins (E-F) using binding agents developed via phage display.
Figure 3 illustrates the relative quantification of the IL8 target protein to its standard protein in co-captured samples. In the co-capture, binding agents towards IL8, APOA1 and IL6 were used, as well as a blank sample. "Mix" is a non-captured sample in a complex background (BSA). "Target" serves as a positive control.

### Examples

### Example 1

### Selection of binding agents

### Set of target proteins

Phage display selections was used to find binding agents having affinity for secretome targets using an in-house scFv library. For the development of binding agents, the following target proteins were used during the selection process: TNF, KLK3, REN, IL6, CRP, EPO, IL8, CLU, APOA1 and CGA. All proteins were produced and purified in-house.

### Biotinylation of target proteins

Each target protein was diluted to a final concentration of 0.25 µg/µl in a 1xPBS solution containing a 10 times molar excess of biotinylation reagent (EZ-Link Sulfo-NHS-LC-Biotin) and incubated for 1 h in a rotomixer at room temperature (RT). After incubation, the target protein was transferred into a pre-hydrated Slide-A-Lyzer^{®} cassette and any remaining air in the cassette was removed. The Slide-A-Lyzer^{®} was then incubated in dialysis buffer (1xPBS) for 2 hours on a magnetic stirrer at RT. The dialysis buffer was replaced after two hours and the Slide-A-Lyzer^{®} cassette was subsequently incubated overnight on a magnetic stirrer at 4 °C. The sample was retrieved from the Slide-A-Lyzer^{®} and transferred to 1.5 ml tubes and the concentration of the sample was determined using absorbance measurements at 280 nm.

### Evaluation of biotinylation

In order to evaluate whether or not biotinylation had been successful, the target protein was subjected to a bead binding test. 100 µl magnetic beads (Dynabeads^{™} M-280 Streptavidin, SA beads) were washed thoroughly in PBST (1xPBS, 0.05% Tween20) and then resuspended in 100 µl PBST. 5 µg of biotinylated protein in 200 µl PBST was then incubated with 25 µl beads for 15 min in a rotomixer. The beads were separated from the supernatant and washed 4 times in 1,000 µl PBST and stored on ice. The supernatant was transferred to a new tube with 25 µl beads and this process was repeated a total of three times. The final supernatant was precipitated by addition of 1 ml of acetone and incubated at -20 °C for 60 min. The supernatant was then removed from the precipitate. The beads from the different fractions and the precipitate were then evaluated using SDS-PAGE to ensure sufficient biotinylation and retrieval.

### Selection - Round 1

### Selection

40 µl SA beads per target protein were washed in PBST and then transferred to a 1.5 ml tube. The SA beads were then incubated in 200 pmol biotinylated target protein diluted in PBST to a total volume of 500 µl for 1 h on a rotomixer at RT. Additionally, 40 µl of beads were washed in PBST and incubated with 800 µl of phage library for 1 h in a rotomixer. The beads, incubated in target protein, were washed in 2x1 ml PBST and the supernatant from the beads incubated in the phage solution was mixed with the washed target protein beads and transferred to 96 well plate. The beads and phages were incubated on a KingFisher^{™} for 3 hours with slow mixing. The beads were then washed in 800 µl PBST 5x1 min with slow mixing and eluted using 500 µl elution buffer (0.25% trypsin, 0.02% Tween20) and 30 min incubation. The eluate was transferred to 1.5 ml tubes and 250 µl aprotinin was added to each tube (0.2 mg/ml).

### Amplification

In order to amplify the eluted phages, 0.6 ml of the eluted solution was transferred to 50 ml of actively growing XL1-blue cells (Agilent Technologies, cat no 200249) and incubated for 30 min at 37 °C. The cells were subsequently spun down, and the cell pellet was resuspended in 2 ml supernatant. The resuspended cells were spread on an agar plate supplemented with tetracycline (10 mg/ml), carbenicillin (100 mg/ml), kanamycin (25 mg/ml) and 1 % glucose and incubated at 37 °C overnight (O/N).

The colonies on the agar plate were resuspended in 2x10 ml 2xYT Broth and OD600 was measured for the cell suspension. 45 ml medium (2xYT Broth, TET10/CARB100/GLU1%) was inoculated with the cell suspension to a final OD600 of 0.15-0.2. The cells were incubated at 37 °C until they reached an OD600 of 0.5. The cells were then infected with M13K07 helper phages and incubated for 1 h at 37 °C. The cells were subsequently pelleted through centrifugation and the medium was discarded. The pellet was resuspended in 1 ml 2xYT Broth and transferred to baffled flask containing 45 ml 2xYT Broth (tetracycline (10 mg/ml), carbenicillin (100 mg/ml), kanamycin (25 mg/ml), and isopropyl β-d-1-thiogalactopyranoside (0.25 mg/ml)) and incubated O/N at 30 °C.

### Precipitation

The culture was transferred to a 50 ml tube and centrifuged at 10000 *g* and the supernatant was transferred to a 50 ml containing 10 ml PEG/NaCl (20% PEG8000, 2.5M NaCl) and incubated on ice for 60 min before being centrifuged at 12000 *g* for 30 min at 4 °C. The supernatant was then discarded, and the pellet was resuspended in 2 ml PBST. The resuspended pellet was centrifuged at 10000 *g* for ten minutes and the supernatant containing the phages was collected in 2 ml tubes.

### Selection - Round 2

### Selection

For the second selection round, 20 µl of SA beads per target protein were washed in PBST and transferred to a 1.5 ml tube and incubated in 100 pmol target protein diluted in PBST to a final volume of 500 µl and incubated on a rotomixer at RT for 1 h. The beads were washed with 2x1 ml PBST and 800 µl of the eluted phages from round 1 were added to the beads. The beads were incubated for 1 h at RT in a rotomixer. The beads were washed in 2x1 ml PBST and transferred to a 96-well plate. The beads and phages were incubated in a KingFisher^{™} for 1.5 hours with slow stirring and then washed 5x1 min with slow stirring in 800 µl PBST. The phages were then eluted with 500 µl elution buffer for 30 min with slow stirring and transferred to 1.5 ml tubes containing 250 µl aprotinin solution.

### Amplification

The phages were amplified by adding 350 µl of the eluate to 10 ml of actively growing (OD600: 0.5-0.7) XL1-blue cells (Agilent Technologies, cat no 200249) in a 50 ml tube and incubated at 37 °C for 30 min. Subsequently 10 ml of 2xYT Broth (TET10/CARB100/GLU1%) was added to the tube and it was incubated for 30 min at 37 °C with shaking at 180 rpm. The cells were then infected with M13K07 helper phages and incubated for 1 h at 37 °C. The cells were then pelleted by centrifugation and the supernatant was discarded. The pellet was resuspended in 50 ml 2xYT Broth
(TET10/CARB100/KAN25/IPTG0.25) and incubated O/N at 37 °C with shaking at 140 rpm.

The phages were precipitated as described above.

### Selection - Round 3

For the third round, 800 µl of the precipitated phages from the second round was mixed with 50 pmol target protein and incubated in a rotomixer at RT for 1 h. Meanwhile, 20 µl SA beads per target protein were washed in 2x1 ml PBST. The target protein-phage suspension was transferred into a 96-well plate together with the beads and incubated in a KingFisher^{™} for 30 min with slow mixing. The beads were subsequently washed with 6x1 min with 800 µl PBST with medium mixing and then eluted over 30 min in elution buffer with slow mixing. The eluates were then transferred to 1.5 ml tubes with 250 µl aprotinin solution.

The phages were amplified as described for round 2 and then precipitated.

### Selection - Round 4

For the final selection round, 800 µl of the precipitated phages from round three were incubated with 10 pmol target protein and then mixed with 20 µl washed beads in a 96-well plate, as described for round three. The mixture was incubated for 30 min with slow mixing in a KingFisher^{™} with slow mixing and the beads were subsequently washed 9x1 min in 800 µl PBST with medium mixing. The phages were eluted in 500 µl elution buffer over 30 min with slow mixing.

The phages from rounds three and four were then amplified as described for round two. 3 ml of each culture were saved for Miniprep purification.

### Miniprep purification

In order to prepare the genetic material for re-cloning into an scFv expression vector, minipreps were performed using the GeneJET Miniprep kit (Thermo Scientific, #K0503) according to the supplier's instructions. The material was eluted in a final volume of 50 µl and the concentration was determined using a NanoPhotometer NP80 (Implen GmbH; München, Germany) and stored at -20 °C.

The genetic material was subsequently cloned into an expression vector for optimized production of scFv fused to a FLAG tag, and transformed for production of scFv. The cells were subsequently grown on agar plates and 47 colonies from round three and 48 colonies from round four were selected for each target protein and transferred to 96-well plates and incubated O/N.

### Evaluation of binding agents

### ELISA evaluation of binding agents

In order to determine which binding agents (in this case scFv fragments) performed the best in terms of signal to noise, all selected binding agents were evaluated against their respective target protein and SA.

A 384-well plate was coated in SA by addition of SA diluted in PBS to a final concentration of 1 mg/ml and incubation O/N at 4 °C. The solution was removed and the plate was subsequently washed in assay buffer (PBS, 0.5% BSA, 0.05% Tween20). Any remaining liquid was removed. The biotinylated target proteins were diluted ten times in assay buffer and were added to half of the wells of the plates. The plate was incubated and subsequently washed in assay buffer. Each clone of the different binding agents was subsequently added to four wells of the plates (two wells with the respective target protein and two wells with SA) and incubated before being washed. HRP-anti-FLAG antibody was added to each well and the plates were incubated before being washed. Substrate was subsequently added to all wells and after quenching, the plates were analyzed through a plate readout at 450 nm.

For each target protein, the binding agents showing the highest signal to noise were selected and the clones (a total number of 384 clones) were sent for DNA sequencing by Eurofin Genomics. All unique clones were subsequently transferred to new 96-well plates and used for further analysis.

### Affinity screening by surface plasmon resonance (SPR)

An initial screening was performed on a BIAcore T200 system for which a CM5 chip with immobilized anti-FLAG antibody (#M8592, Sigma-Aldrich) was used for the analysis. For the analysis, the chip was flowed with target protein specific scFv from the bacterial supernatants to saturate the surface of the chip by binding to the FLAG tag present on all scFvs. The chip was thereafter flowed with 50 nM of non-biotinylated target protein. The chip was regenerated using a 10 mM glycine HCl buffer with a pH of 2.1. The binding agents which bound to the target protein with the highest affinity were selected for a more thorough kinetics analysis.

The same workflow as described above was used for determining the kinetic constants for the binding agents, but instead of flowing the chip with one concentration of target protein a four-fold dilution curve ranging from 0.3 nM to 80 nM was flowed across the chip. The kinetic constants were determined by fitting a 1:1 Langmuir binding model to the response curves.

### Evaluation of target specificity

To assess the specificity of the binding agents an ELISA was used. The plates were prepared as described above, but instead of evaluating each binder against its respective target protein and SA, the binding agents were evaluated against all target proteins used in the selection process. Only binding agents showing high specificity towards their target protein were selected.

### Screening for protein fragment binding agents

In order to assess the ability of the binding agents to bind recombinant protein standards, the affinity of the binding agents to different protein fragments from each respective protein target was evaluated by SPR as described above. Binding agents that were able to bind both target protein and recombinant protein fragments with similar affinity were used for co-capture of target protein and protein fragments for subsequent mass spectrometry (MS) readout.

### Capture for MS readout

### Capturing and digestion for MS readout

Protein A-coupled beads were coated with the obtained binding agents for the targets APOA1, IL6 and IL8. 15 µl Protein A-coupled beads (Pierce/Thermo Fisher) per target protein were washed three times in 500 µl washing buffer (0.1 µg/µl casein hydrolysate, 0.3% (w/v) Chaps, PBS). The washing buffer was removed, and the beads were resuspended in 30 µl binding buffer (0.5 µg/µl casein hydrolysate, 0.3% (w/v) Chaps, 1xPBS). The beads were subsequently incubated with 1.2 µg of the respective binding agent in individual tubes and diluted to a final volume of 100 µl in binding buffer. The samples were incubated for 30 min at RT in a rotomixer. The supernatant was removed, and the beads were resuspended in 30 µl binding buffer. Beads coated with binding agent, i.e. "binding agent coated beads" had thus been obtained.

For the capture of target proteins and stable isotope labeled (Protein Recombinant Isotope Standard (PRecIS)) protein fragments of APOA1, IL6 and IL8, 10 µl of each of the binding agent coated beads were transferred to tubes with 20 µl pre-aliquoted binding buffer in duplicates. The amount of added binding agents was adjusted based on the endogenous amount of target proteins in order to decrease the dynamic range of captured molecules. The relative concentration between APOA1, IL6 and IL8 binding agents was 10:1:1 Additionally, a negative sample consisting of washed bare beads was prepared.

A mixture of the target proteins and the corresponding isotope-labelled standard proteins (PRecIS) in binding buffer (Table 2) was added to each tube. The samples were diluted to a final volume of 200 µl in binding buffer and incubated for 1 h at RT in a rotomixer. The supernatant was subsequently removed. The beads were thereafter washed twice in 500 µl 1x wash buffer and the supernatant was discarded. 10 µl of 10 mM dithiothreitol in 50 mM ammonium bicarbonate was added to the beads and the samples were incubated at 56 °C for 30 min. 1 µl of 500 mM 2-chloroacetamide was subsequently added to the tubes and the samples were incubated at RT, shielded from light, for 30 min. SOLu-Trypsin (#EMS0004, Sigma Aldrich) was thereafter added to the samples to a final amount of 0.5 µg and the samples were incubated O/N at 37 °C. The digestion was quenched by addition 10% formic acid (FA) to a final concentration of 0.5% FA and the supernatant was transferred to a HPLC vial for analysis by MS.

**Table 2. Target protein and standard isotope-labelled protein mixture composition.**

| **Protein** | **Concentration (µM)** |
|---|---|
| APOA1 | 10 |
| IL6 | 1 |
| IL8 | 1 |
| PRecIS APOA1 | 10 |
| PRecIS IL6 | 1 |
| PRecIS IL8 | 1 |

### Mass spectrometry analysis

For the MS analysis of captured proteins digested into peptides, samples were analyzed using a TSQ Altis (Thermo Fisher) coupled to a Dionex Ultimate 3000 liquid chromatography (LC) system (Thermo Fisher) equipped with a cartridge type trap column (160434, Thermo Fisher) and a 15 cm analytical EASY-spray column (ES806A, Thermo Scientific) with a mobile phase consisting of solvent A (3% acetonitrile (ACN), 0.1% FA) and solvent B (95% ACN, 0.1% FA). For the analysis, 10 µl of each sample was loaded onto the column and separated over a 15 min long run with a flow rate of 3 µl/min. The gradient used for the separation of peptides was as follows: 1% solvent B for 0.75 min, 1-30% solvent B for 9.25 min, 30-95% solvent B for 0.1 min, followed by a cycling between 0% and 95% solvent B three times during 3 min, finally followed by a re-equilibration of the column at 1% solvent B for 1.5 min. The MS was operated in an unscheduled SRM mode, monitoring the 10 most intense product ions for all tryptic peptides present both in the PRecIS protein fragments and the target proteins, with a dwell time of 0.5 ms. All data analysis was performed in Skyline Targeted Mass Spec Environment (University of Washington).

### Example 2

### Determination of affinity to target proteins and corresponding standard proteins

A set of eight target proteins were selected for assay development based on clinical relevance and reference concentration in plasma. They are listed in Table 3, sorted by increasing plasma concentration reference range for the corresponding protein.

**Table 3. Proteins selected for recombinant antibody generation.**

| **Protein** | **Modification during selection** | **Reference concentration** |
|---|---|---|
| 1. TNF | chemically biotinylated | 14 ng/l |
| 2. IL6 | chemically biotinylated | 5.1 ng/l |
| 3. IL8 | chemically biotinylated | 6 ng/l |
| 4. KLK3 | chemically biotinylated | NA |
| 5. CRP | chemically biotinylated | 1.6 mg/l |
| 6. REN | chemically biotinylated | 690 ng/l |
| 7. CLU | chemically biotinylated | 110 mg/l |
| 8. APOA1 | chemically biotinylated | 1.4 g/l |

Phage display selections were performed to find binding agents for the target proteins. An in-house scFv library was used as described in Example 1. The biotinylated recombinant proteins of Table 3 were subjected to western blot analysis in order to verify their molecular weight prior to phage display selection.

Successfully biotinylated target proteins were bound to magnetic streptavidin beads and used for phage display selection in a four round setup. Selected binding phages (successfully enriched clones) were eluted using trypsin/aprotinin and re-cloned into a scFv expression vector.

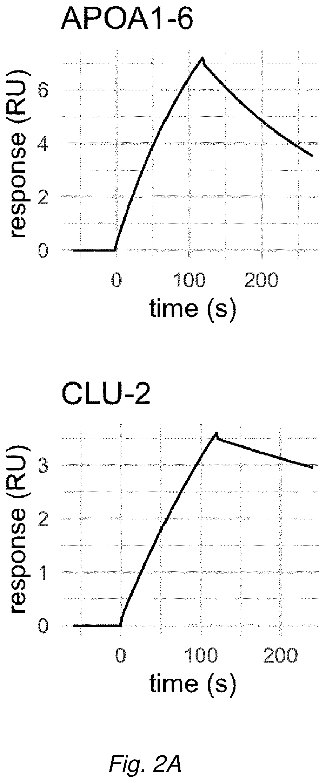

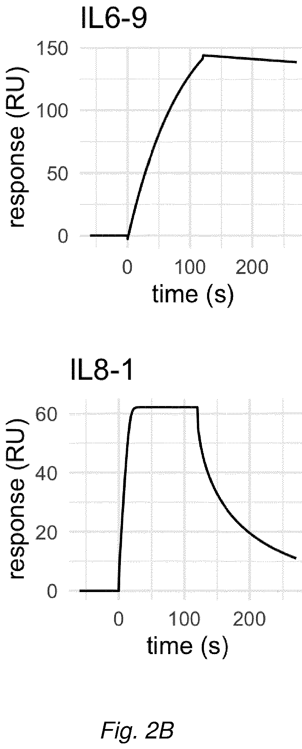

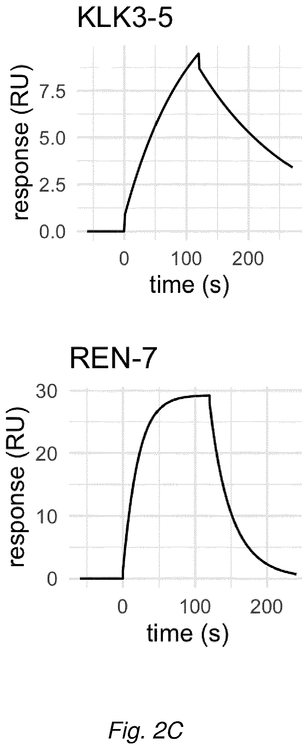

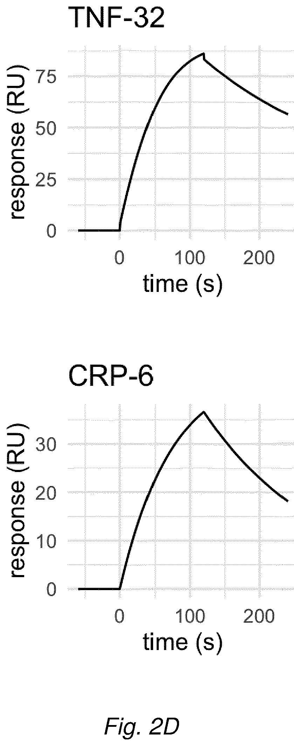

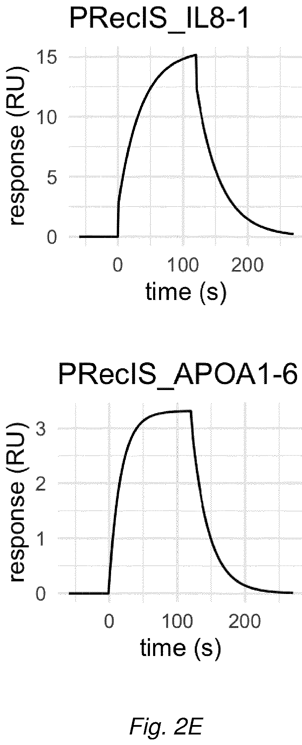

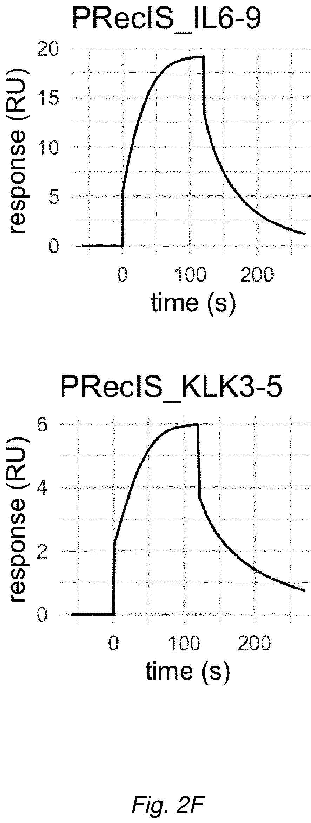

Binding of the scFv clones from the phage selection was verified by ELISA. Positive scFv clones were selected for surface plasmon resonance (SPR) measurement towards their target protein. Furthermore, those clones were in parallel screened towards isotope-labelled internal standard proteins corresponding to their target protein. The identity of the scFv binding agents and their binding constants were determined using a kinetic SPR setup (Table 4). Figure 2A-D shows SPR for scFv binding agents towards eight target proteins (full-length proteins) after the final round of selection. Figure 2E-F shows SPR for four scFv binders successfully binding to a corresponding PRecIS standard protein version of the full-length protein.

### Results

In total, six scFv binding agents towards IL8, eight towards IL6, three towards KLK3 and two binding agents towards APOA1 that successfully recognized the two different forms of their target were obtained. That is, the obtained scFv binding agents recognized their target standard protein as well as its corresponding isotope-labelled internal standard protein.

The affinity of the binding agent denoted "IL8-12" towards a corresponding isotope-labelled standard protein was determined to be 78 nM, while the affinity of the same binding agent towards the IL8 target protein was more than 3 times lower, determined to be 250 nM (Table 4, entry 5).

**Table 4. Binding agents recognizing both a protein and its corresponding fragment**

| **scFv clone name** | **Antigen** | **Ka (1/Ms)** | **Kd (1/s)** | **KD (M)** | **Ka (1/Ms)** | **Kd (1/s)** | **KD (M)** | **Kd ratio (Target: PRecIS)** | **KD ratio (Target: PRecIS)** |
|---|---|---|---|---|---|---|---|---|---|
| | | **Target** | **Target** | **Target** | **PRecIS** | **PRecIS** | **PRecIS** | | |
| AB-IL8-1 | IL8 | 4.9x10⁶ | 4.1x10⁻² | 8.4x10⁻⁹ | 4.0x10⁴ | 1.3x10⁻² | 3.1x10⁻⁷ | 3.15 | 0.03 |
| AB-IL8-5 | IL8 | 1.8x10³ | 1.2x10⁻³ | 6.7x10⁻⁷ | 1.5x10⁴ | 4.3x10⁻³ | 2.8x10⁻⁷ | 0.28 | 2.39 |
| AB-IL8-7 | IL8 | 4.0x10³ | 1.8x10⁻³ | 4.4x10⁻⁷ | 3.1x10⁴ | 1.0x10⁻² | 5.1x10⁻⁷ | 0.18 | 0.86 |
| AB-IL8-9 | IL8 | 8.2x10⁴ | 1.4x10⁻² | 1.7x10⁻⁷ | 3.1x10⁴ | 4.1x10⁻³ | 1.3x10⁻⁷ | 3.41 | 1.31 |
| AB-IL8-12 | IL8 | 3.7x10⁴ | 9.4x10⁻³ | 2.5x10⁻⁷ | 1.1x10⁹ | 8.5x10¹ | 7.8x10⁻⁸ | 1.1x10⁻⁴ | 3.21 |
| AB-IL8-14 | IL8 | 3.4x10⁵ | 1.0x10⁻² | 3.0x10⁻⁸ | 1.6x10⁴ | 7.8x10⁻³ | 4.9x10⁻⁷ | 1.28 | 0.06 |
| AB-IL6s-3 | IL6 | 4.0x10⁵ | 9.8x10⁻⁵ | 2.5x10⁻¹⁰ | 1.0x10⁵ | 6.0x10⁻³ | 5.9x10⁻⁸ | 0.02 | 0.00 |
| AB-IL6s-4 | IL6 | 1.4x10⁵ | 4.8x10⁻⁴ | 3.5x10⁻⁹ | 2.6x10⁵ | 1.5x10⁻³ | 5.8x10⁻⁸ | 0.32 | 0.06 |
| AB-IL6s-6 | IL6 | 1.7x10⁵ | 5.0x10⁻⁴ | 3.0x10⁻⁹ | 9.9x10⁴ | 4.9x10⁻³ | 4.9x10⁻⁸ | 0.10 | 0.06 |
| AB-IL6s-9 | IL6 | 3.7x10⁵ | 5.5x10⁻⁴ | 1.5x10⁻⁹ | 1.1x10⁵ | 3.7x10⁻³ | 3.5x10⁻⁸ | 0.15 | 0.04 |
| AB-IL6s-10 | IL6 | 2.1x10⁵ | 2.2x10⁻⁴ | 1.0 x10⁻⁹ | 9.9x10⁴ | 4.4x10⁻³ | 4.4x10⁻⁸ | 0.05 | 0.02 |
| AB-IL6s-12 | IL6 | 5.7x10⁴ | 2.6x10⁻⁴ | 4.5x10⁻⁹ | 7.8x10⁴ | 4.2x10⁻³ | 5.4x10⁻⁸ | 0.06 | 0.08 |
| AB-IL6s-16 | IL6 | 2.1x10⁵ | 5.8x10⁻⁴ | 2.7x10⁻⁹ | 1.1x10⁵ | 4.6x10⁻³ | 4.1x10⁻⁸ | 0.13 | 0.07 |
| AB-IL6s-18 | IL6 | 1.8x10⁵ | 6.5x10⁻⁴ | 3.6x10⁻⁹ | 9.6x10⁴ | 3.6x10⁻³ | 3.8x10⁻⁸ | 0.18 | 0.09 |
| AB-KLK3-1 | KLK3 | 2.3x10⁵ | 4.2x10⁻³ | 1.9x10⁻⁸ | 1.2x10⁹ | 9.0x10¹ | 7.6x10⁻⁸ | 4.7x10⁻⁵ | 0.25 |
| AB-KLK3-5 | KLK3 | 2.9x10⁴ | 5.9x10⁻³ | 2.0x10⁻⁷ | 7.5x10³ | 5.3x10⁻³ | 7.1x10⁻⁷ | 1.11 | 0.28 |
| AB-KLK3-15 | KLK3 | 2.4x10⁵ | 1.1x10⁻² | 4.5x10⁻⁸ | 1.1x10⁵ | 1.2x10⁻² | 1.1x10⁻⁷ | 0.92 | 0.41 |
| AB-KLK3-1 | KLK3 | 2.3x10⁵ | 4.2x10⁻³ | 1.9x10⁻⁸ | 8.5x10⁴ | 1.0x10⁻² | 1.2x10⁻⁷ | 0.42 | 0.16 |
| AB-KLK3-5 | KLK3 | 2.9x10⁴ | 5.9x10⁻³ | 2.0x10⁻⁷ | 3.8x10⁴ | 3.4x10⁻² | 9.1x10⁻⁷ | 0.17 | 0.22 |
| AB-KLK3-15 | KLK3 | 2.4x10⁵ | 1.1x10⁻² | 4.5x10⁻⁸ | 7.4x10⁴ | 8.0x10⁻³ | 1.1x10⁻⁷ | 1.38 | 0.41 |
| AB-APOA1-6 | APOA1 | 2.5x10⁴ | 4.1x10⁻³ | 1.7x10⁻⁷ | 8.0x10⁴ | 3.8x10⁻² | 4.8x10⁻⁷ | 0.11 | 0.35 |
| AB-APOA1-25 | APOA1 | 4.2x10⁶ | 3.4x10⁻¹ | 7.9x10⁻⁸ | 1.1x10⁵ | 4.3x10⁻² | 4.0x10⁻⁷ | 7.91 | 0.20 |

### Example 3

### Co-capture of target proteins and corresponding standard proteins in a complex background

### Preparation of mixture of target proteins and corresponding standard proteins

A pool of target proteins and corresponding standard proteins was established by pooling 10 pmol secretome APOA1 (30736 Da), 1 pmol secretome IL8 (11766 Da) and 1 pmol secretome IL6 (23470 Da) into a buffer comprising 1% casein hydrolysate, 0.3% *(w*/*v)* Chaps, and 1xPBS. To this mixture, the corresponding standard proteins were pooled in equimolar amounts (10 pmol PRecIS APOA1, HPRR3450265; 1 pmol PRecIS IL6, HPRR330007; and 1 pmol PRecIS IL8, HPRR2700195; Edfors et al (2019), J Proteome Res 18(7):2706-2718).

In addition, a sample of the mixture was spiked into a complex background of bovine serum albumin (BSA). This mixture served as a baseline for the following experimental procedure.

### Preparation of beads comprising binding agent

60 µl Protein A (Thermo Scientific) magnetic beads were washed three times with 500 µl wash buffer (0.1 µg/µl casein hydrolysate, 0.3% *(w*/*v)* Chaps, 1xPBS). Supernatant was removed after first collecting the beads using a magnetic stand and beads were re-dissolved in 120 µl binding buffer (0.5 µg/µl casein hydrolysate, 0.3% *(w*/*v)* Chaps, 1xPBS). A total of 30 µl beads were split into four separate tubes (for the binding agents towards IL6, IL8, APOA1 and negative control, respectively). The negative control consisted of bare beads with no binding agent. A total of 1.2 µg binding agent (anti-APOA1, anti-IL6 and anti-IL8 scFv (denoted IL8-5)), respectively, was immobilized onto the Protein A magnetic beads, by incubation for 30 minutes. Excess buffer was removed after first collecting the beads using a magnetic stand.

### Co-capturing

10 µl of the prepared pool of target proteins and corresponding standard proteins in casein background were added to each respective tube of beads comprising binding agent, together with 190 µl binding buffer. Target proteins and corresponding standard proteins were captured by incubation for 1 h at RT on a rotor mixer. The supernatant was then removed, and the beads were washed once with 500 µl 1x wash buffer (0.01 µg/µl casein hydrolysate, 0.03% *(w*/*v)* Chaps, 0.1xPBS) and once with 500 µl 0.1x wash buffer.

### Digestion and analysis

Captured proteins were reduced on the beads following the addition of 10 µl 10 mM DTT in 50 mM ammonium bicarbonate and incubated for 30 minutes at 56 °C. The proteins were alkylated following addition of 2-chloroacetamide (CAA) to a final concentration of 50 mM and incubated in the dark for 30 minutes at RT. Digestion was performed by the addition of 0.5 µg trypsin to the sample. The sample was incubated overnight at 37 °C. The reaction was quenched with formic acid to a final concentration of 0.5 % (v/v). The beads were removed using a magnet, and the peptide digest was analyzed using LC-MS/SRM after addition on BSA.

### Results

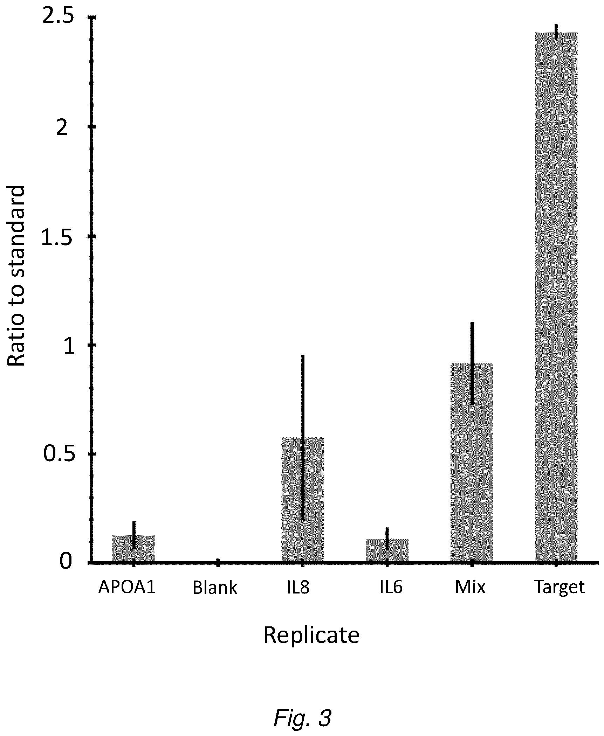

The mass spectrometry read-out revealed that the binding agent anti-IL8 scFv successfully co-captures the target protein together with its corresponding standard protein (PRecIS) (Figure 3).

Figure 3 is a bar plot over the relative ratios between heavy and light peptides from IL8 (ratio-to-standard between endogenous IL8 and PRecIS IL8). The ratio was quantified for the samples of co-captured target and standard proteins (that was co-captured using the different scFv binding agents anti-APOA1, anti-IL8 and anti-IL6), after dilution of the samples in BSA. The sample that was subjected to the negative control (bare beads) was also quantified after addition of BSA. BSA was added to form a complex background.

In addition, the ratio between target protein and corresponding PRecIS was quantified for the pool of target proteins (APOA1, IL6 and IL8 and corresponding PRecIS) diluted in BSA, and quantified without capturing ("Mix"). The "Mix" can be considered as the baseline for this experimental procedure. The same ratio was quantified for a non-diluted pool of the target proteins APOA1, IL6 and IL8 and corresponding PRecIS without capturing ("Target"). The "Target" represents a pool of non-diluted recombinant proteins and serves as a positive control.

Each protein name on the x-axis in Figure 3 (APOA1, IL8, IL6) represents quantification after the co-capturing using one scFv corresponding to that target protein. The blank sample represents quantification after the co-capturing using bare beads (no scFv). "Mix" represents the target mixture after spiking it into a complex background (BSA) and "Target" represents undiluted protein mixture. The y-axis in Figure 3 represents the relative ratio between heavy (isotope labeled) and light (unlabeled) peptide, quantified by a targeted proteomics read out performed by Selective Reaction Monitoring.

### Conclusion

This experiment shows that IL8 and PRecIS-IL8 were specifically captured in the co-capturing using an anti-IL8 scFv, compared to anti-APOA1, anti-IL6 and the negative control. Thus, binding agents generated towards full length protein sequences can co-capture proteins with identical and or similar epitopes presented on their surface. The difference in affinity of the generated scFv binding agent for the target protein and the internal standard, respectively, resulted in less effective capturing of the PRecIS relative to its full-length recombinant protein (secretome). However, it does not matter which of the target protein or the PRecIS that binds with higher affinity, as long as one knowns the difference in affinity. As discussed in the general sections of the disclosure, this effect can be accounted for if the difference in efficiency is known. To conclude, such pull-down or co-capturing experiments can therefore successfully be used for quantitative proteomics.

## Claims

1. A method for measuring the amount of a target protein in body fluid, the method comprising the following consecutive steps:
- preparing a sample suspected to comprise said target protein and comprising a known amount of an isotope-labelled internal standard protein, said standard protein consisting of a fragment of said target protein,
- bringing said sample into contact with a solid support comprising a binding agent,
- washing said solid support to remove unbound members of the sample,
- digesting said target protein and said standard protein to provide a digested sample,
- subjecting said digested sample to mass spectrometry, and
- determining the amount of said target protein in said sample by comparison with said standard, wherein
said binding agent is capable of binding an epitope present in both said target protein and said standard protein, wherein
said measuring comprises measuring the amount of at least two target proteins, such as three target proteins, such as four target proteins, such as five target proteins, such as six target proteins, such as seven target proteins, such as eight target proteins, such as nine target proteins, such as ten target proteins, and wherein the plurality of target proteins are measured in parallel (i.e. multiplex mode).

2. The method according to claim 1, wherein said target proteins are actively secreted proteins.

3. The method according to any one of the preceding claims, wherein said target proteins are selected from the group consisting of a cytokine, a chemokine, an interleukin, an interferon, a hormone, a neuropeptide, a growth factor, a receptor, a protein involved in transport, a protein involved in development, an enzyme, an enzyme inhibitor, a protein involved in the immune system, a protein involved in coagulation, a protein involved in the complement pathway, an acute phase protein and a cell adhesion protein.

4. The method according to any one of the preceding claims, wherein said solid support is selected from the group consisting of a bead, such as a magnetic bead, and a column.

5. The method according to any one of the preceding claims, wherein said digestion is carried out by means of a proteolytic enzyme.

6. The method according to any one of the preceding claims, wherein the digested sample comprises at least one isotopically labeled standard peptide consisting of between 6 and 25 amino acids.

7. The method according to any one of the preceding claims, wherein said standard protein comprises at least two cleavage sites for said proteolytic enzyme.

8. The method according to any one of the preceding claims, wherein said standard protein is labelled with at least one isotope selected from the group consisting of ¹⁵N, ¹³C and/or ¹⁸O.

9. The method according to any one of the preceding claims, wherein said binding agent is an antibody or an antibody fragment.

10. The method according to any one of the preceding claims, wherein said at least one target protein suspected to be present in said sample, is present in said sample in a concentration of between 10⁻⁴ and 10⁻¹⁰ M**,** such as between 10⁻⁶ and 10⁻⁷ M.

11. The method according to any one of the preceding claims, wherein said target protein and said standard protein bind to the binding agent with comparable affinity, at a ratio of K_{D} values such as 1:1, such as 1:2, such as 1:3, such as 1:4, such as 1:5, such as 1:6, such as 1:7, such as 1:8, such as 1:9, such as 1:10, such as 1:11, such as 1:12, such as 1:13, such as 1:14, such as 1:15.

12. The method according to any one of the preceding claims, wherein said epitope comprises at least 4 amino acids, such as 5 amino acids, such as 6 amino acids, such as 7 amino acids, such as 8 amino acids, such as 9 amino acids, such as 10 amino acids, such as 11 amino acids, such as 12 amino acids, such as 13 amino acids, such as 14 amino acids, such as 15 amino acids.

13. The method according to any one of the preceding claims, wherein said method is preceded by a step of approximation of the amount of target protein by establishing a standard curve, such as a forward standard curve or a reverse standard curve.

## Patentansprüche

1. Verfahren zur Messung der Menge eines Zielproteins in einer Körperflüssigkeit, wobei das Verfahren nacheinander die folgenden Schritte umfasst:
- Herstellen einer Probe, die mutmaßlich das Zielprotein umfasst, wobei sie eine bekannte Menge eines isotopenmarkierten internen Standardproteins umfasst, wobei das Standardprotein aus einem Fragment des Zielproteins besteht,
- Inkontaktbringen der Probe mit einem festen Träger, der ein bindendes Mittel umfasst,
- Waschen des festen Trägers, um ungebundene Bestandteile der Probe zu entfernen,
- Verdau des Zielproteins und des Standardproteins, um eine verdaute Probe bereitzustellen,
- Einwirkenlassen von Massenspektrometrie auf die verdaute Probe, und
- Bestimmen der Menge des Zielproteins in der Probe durch Vergleich mit dem Standard, wobei das bindende Mittel dazu befähigt ist, an ein Epitop zu binden, das sowohl in dem Zielprotein als auch in dem Standardprotein vorliegt, wobei
wobei das Messen ein Messen der Menge an mindestens zwei Zielproteinen, wie etwa drei Zielproteinen, wie etwa vier Zielproteinen, wie etwa fünf Zielproteinen, wie etwa sechs Zielproteinen, wie etwa sieben Zielproteinen, wie etwa acht Zielproteinen, wie etwa neun Zielproteinen, wie etwa zehn Zielproteinen umfasst, und wobei die Mehrzahl an Zielproteinen parallel (d.h. Im Multoplex-Modus) gemessen werden.

2. Verfahren gemäß Anspruch 1, wobei es sich bei den Zielproteinen um aktiv sezernierte Proteine handelt.

3. Verfahren gemäß einem beliebigen der vorhergehenden Ansprüche, wobei die Zielproteine aus der Gruppe ausgewählt sind, die aus einem Zytokin, einem Chemokin, einem Interleukin, einem Interferon, einem Hormon, einem Neuropeptid, einem Wachstumsfaktor, einem Rezeptor, einem Protein, welches am Transport beteiligt ist, einem Protein, welches an der Entwicklung beteiligt ist, einem Enzym, einem Enzymhemmer, einem Protein, welches am Immunsystem beteiligt ist, einem Protein, welches an der Gerinnung beteiligt ist, einem Protein, welches am Komplement-Stoffwechselweg beteiligt ist, einem Akutphasenprotein und einem Zelladhäsionsprotein besteht.

4. Verfahren gemäß einem beliebigen der vorhergehenden Ansprüche, wobei der feste Träger aus der Gruppe ausgewählt ist, die aus einer Perle, wie etwa einer magnetischen Perle, und einer Säule besteht.

5. Verfahren gemäß einem beliebigen der vorhergehenden Ansprüche, wobei der Verdau mittels eines proteolytischen Enzyms durchgeführt wird.

6. Verfahren gemäß einem beliebigen der vorhergehenden Ansprüche, wobei die verdaute Probe mindestens ein isotopenmarkiertes Standardpeptid umfasst, das aus 6 bis 25 Aminosäuren besteht.

7. Verfahren gemäß einem beliebigen der vorhergehenden Ansprüche, wobei das Standardprotein mindestens zwei Spaltungsstellen für das proteolytische Enzym umfasst.

8. Verfahren gemäß einem beliebigen der vorhergehenden Ansprüche, wobei das Standardprotein mit mindestens einem Isotop markiert ist, das aus der Gruppe ausgewählt ist, welche aus ¹⁵N, ¹³C und/oder ¹⁸O besteht.

9. Verfahren gemäß einem beliebigen der vorhergehenden Ansprüche, wobei es sich bei dem bindenden Mittel um einen Antikörper oder ein Antikörperfragment handelt.

10. Verfahren gemäß einem beliebigen der vorhergehenden Ansprüche, wobei mindestens ein Zielprotein, von dem vermutet wird, dass es in der Probe vorliegt, in einer Konzentration zwischen 10⁻⁴ und 10⁻¹⁰ M, wie etwa zwischen 10⁻⁶ und 10⁻⁷ M, in der Probe vorliegt.

11. Verfahren gemäß einem beliebigen der vorhergehenden Ansprüche, wobei das Zielprotein und das Standardprotein mit einer vergleichbaren Affinität an das bindende Mittel binden, wobei das Verhältnis der KD-Werte etwa 1:1, etwa 1:2, etwa 1:3, etwa 1:4, etwa 1:5, etwa 1:6, etwa 1:7, etwa 1:8, etwa 1:9, etwa 1:10, etwa 1:11, etwa 1:12, etwa 1:13, etwa 1:14, etwa 1:15 beträgt.

12. Verfahren gemäß einem beliebigen der vorhergehenden Ansprüche, wobei das Epitop mindestens 4 Aminosäuren, wie etwa 5 Aminosäuren, wie etwa 6 Aminosäuren, wie etwa 7 Aminosäuren, wie etwa 8 Aminosäuren, wie etwa 9 Aminosäuren, wie etwa 10 Aminosäuren, wie etwa 11 Aminosäuren, wie etwa 12 Aminosäuren, wie etwa 13 Aminosäuren, wie etwa 14 Aminosäuren, wie etwa 15 Aminosäuren umfasst.

13. Verfahren gemäß einem beliebigen der vorhergehenden Ansprüche, wobei dem Verfahren ein Schritt der näherungsweisen Bestimmung der Menge an Zielprotein durch Erstellen einer Standardkurve, wie etwa einer fortschreitenden Standardkurve oder einer Umkehr-Standardkurve, vorangeht.

## Revendications

1. Procédé de mesure de la quantité d'une protéine cible dans un fluide corporel, le procédé comprenant les étapes consécutives suivantes :
- préparation d'un échantillon suspecté de comprendre ladite protéine cible et comprenant une quantité connue d'une protéine étalon interne marquée par un isotope, ladite protéine étalon étant constituée d'un fragment de ladite protéine cible,
- mise en contact dudit échantillon avec un support solide comprenant un agent liant,
- lavage dudit support solide pour éliminer des éléments non liés de l'échantillon,
- digestion de ladite protéine cible et de ladite protéine étalon pour fournir un échantillon digéré,
- soumission dudit échantillon digéré à une spectrométrie de masse, et
- détermination de la quantité de ladite protéine cible dans ledit échantillon par comparaison avec ledit étalon, dans lequel
ledit agent de liaison est capable de se lier à un épitope présent à la fois dans ladite protéine cible et ladite protéine étalon, dans lequel
ladite mesure comprend la mesure de la quantité d'au moins deux protéines cibles, telles que trois protéines cibles, telles que quatre protéines cibles, telles que cinq protéines cibles, telles que six protéines cibles, telles que sept protéines cibles, telles que huit protéines cibles, telles que neuf protéines cibles, telles que dix protéines cibles, et dans lequel la pluralité de protéines cibles sont mesurées en parallèle (c.-à-d. mode multiplex).

2. Procédé selon la revendication 1, dans lequel lesdites protéines cibles sont des protéines sécrétées activement.

3. Procédé selon l'une quelconque des revendications précédentes, dans lequel lesdites protéines cibles sont choisies dans le groupe constitué par une cytokine, une chimiokine, une interleukine, un interféron, une hormone, un neuropeptide, un facteur de croissance, un récepteur, une protéine impliquée dans le transport, une protéine impliquée dans le développement, une enzyme, un inhibiteur enzymatique, une protéine impliquée dans le système immunitaire, une protéine impliquée dans la coagulation, une protéine impliquée dans la voie du complément, une protéine de phase aiguë et une protéine d'adhésion cellulaire.

4. Procédé selon l'une quelconque des revendications précédentes, dans lequel ledit support solide est choisi dans le groupe constitué par une bille, telle qu'une bille magnétique, et une colonne.

5. Procédé selon l'une quelconque des revendications précédentes, dans lequel ladite digestion est effectuée au moyen d'une enzyme protéolytique.

6. Procédé selon l'une quelconque des revendications précédentes, dans lequel l'échantillon digéré comprend au moins un peptide étalon marqué isotopiquement constitué d'entre 6 et 25 acides aminés.

7. Procédé selon l'une quelconque des revendications précédentes, dans lequel ladite protéine étalon comprend au moins deux sites de clivage pour ladite enzyme protéolytique.

8. Procédé selon l'une quelconque des revendications précédentes, dans lequel ladite protéine étalon est marquée par au moins un isotope choisi dans le groupe constitué par ¹⁵N, ¹³C et/ou ¹⁸O.

9. Procédé selon l'une quelconque des revendications précédentes, dans lequel ledit agent de liaison est un anticorps ou un fragment d'anticorps.

10. Procédé selon l'une quelconque des revendications précédentes, dans lequel ladite au moins une protéine cible suspectée d'être présente dans ledit échantillon, est présente dans ledit échantillon à une concentration comprise entre 10⁻⁴ et 10⁻¹⁰ M, telle qu'entre 10⁻⁶ et 10⁻⁷ M.

11. Procédé selon l'une quelconque des revendications précédentes, dans lequel ladite protéine cible et ladite protéine étalon se lient à l'agent de liaison avec une affinité comparable, à raison d'un rapport de valeurs K_{D} tel que 1:1, tel que 1:2, tel que 1:3, tel que 1:4, tel que 1:5, tel que 1:6, tel que 1:7, tel que 1:8, tel que 1:9, tel que 1:10, tel que 1:11, tel que 1:12, tel que 1:13, tel que 1:14, tel que 1:15.

12. Procédé selon l'une quelconque des revendications précédentes, dans lequel ledit épitope comprend au moins 4 acides aminés, tels que 5 acides aminés, tels que 6 acides aminés, tels que 7 acides aminés, tels que 8 acides aminés, tel que 9 acides aminés, tels que 10 acides aminés, tels que 11 acides aminés, tels que 12 acides aminés, tels que 13 acides aminés, tels que 14 acides aminés, tels que 15 acides aminés.

13. Procédé selon l'une quelconque des revendications précédentes, dans lequel ledit procédé est précédé par une étape d'approximation de la quantité de protéine cible en établissant une courbe étalon, telle qu'une courbe étalon avant ou une courbe étalon inverse.
